# EUROPEAN PATENT APPLICATION

(11) **EP 4 523 680 A1**
(43) Date of publication of application: **19.03.2025**
(21) Application number: 23803801.2
(22) Date of filing: 09.05.2023
(51) Int. Cl.: A61K 9/08, A61K 47/18, A61K 47/26, A61K 47/02, A61K 9/19, A61K 38/48, A61P 7/02

(54) **NOVEL LIQUID FORMULATION FOR PLASMA PROTEIN**

(30) Priority: 10.05.2022 KR 20220057110
(71) Applicant: GREEN CROSS CORPORATION, Yongin-si, Gyeonggi-do 16924 (KR)
(72) Inventor: KIM, Miroo, Yongin-si Gyeonggi-do 16924 (KR); LEE, Hyejin, Yongin-si Gyeonggi-do 16924 (KR); SON, Jaewoon, Yongin-si Gyeonggi-do 16924 (KR); LIM, Jungae, Yongin-si Gyeonggi-do 16924 (KR); LEE, Eun Jeong, Yongin-si Gyeonggi-do 16924 (KR); CHOI, Hyemin, Yongin-si Gyeonggi-do 16924 (KR); NAM, Hyunja, Yongin-si Gyeonggi-do 16924 (KR)
(74) Representative: Mewburn Ellis LLP
(86) International application number: PCT/KR2023/006251
(87) International publication number: WO 2023/219378

(57) **Abstract**

The present invention relates to a pharmaceutical formulation of plasma protein, specifically ADAMTS-13 protein, and a composition for preventing or treating thrombotic disease comprising the same. The pharmaceutical formulation of the present invention significantly improves the stability of plasma protein whose pharmacological activity and quality deteriorates during long term storage due to its high risk of being contaminated and denatured immediately after separation and purification from blood, and in particular, is capable of maintaining the colloidal stability, refrigeration stability, purity, and inhibition of aggregation of the plasma protein at high levels, and allows the cake appearance after lyophilization to be maintained well for a long period of time. Accordingly, the pharmaceutical formulation of the present invention may be advantageously used to maintain and store ADAMTS-13 protein, which is an important therapeutic resource for use for various thrombotic diseases, for a long period of time without loss of the therapeutically effective amount of the protein.

## Description

### Technical Field

The present invention relates to a liquid formulation for a plasma protein, specifically ADAMTS-13 protein.

### Background Art

In the development of protein drugs, it is an important issue to develop an efficient lyophilized formulation to ensure sufficient storage stability for proteins having low stability. It is necessary to store a formulation for a certain period of time in a liquid state before the lyophilization process starts. To prevent quality deterioration during this storage period, it is necessary to ensure stability to some extent even in a liquid formulation.

Excipients that are used in lyophilized formulations are classified into two types, crystalline and amorphous, depending on the properties of the material. Crystalline excipients serve as bulking agents in lyophilized formulations and provide mechanical strength to lyophilized cakes, making the cakes more robust. Typical crystalline excipients include, for example, mannitol and glycine. On the other hand, amorphous excipients serve as protein stabilizers in lyophilized formulations, exist adjacent to proteins, and act to stabilize the proteins from stress generated during the lyophilization process.

Meanwhile, sodium chloride, which is mainly used in protein drug formulations, may exist in two forms, amorphous and crystalline, depending on the concentration added. In general, it has been reported that when NaCl is added at a concentration of 150 mM or less, it is mainly in an amorphous state, and when it is added at a concentration of 200 mM or more, it is mostly crystallized.

The stabilizer in a lyophilized formulation protects and stabilizes the protein from stress generated during the lyophilization process, and also contributes to protein stabilization during storage and reconstitution after completion of drying. Therefore, in particular, in protein formulations scheduled for long-term storage by lyophilization, the search for optimal stabilizer components and the optimal content ratio between them is as important as the discovery of new pharmacological ingredients in terms of the value and utility of proteins as therapeutic resource.

Throughout the specification, a number of publications and patent documents are referred to and cited. The disclosure of the cited publications and patent documents is incorporated herein by reference in their entirety in order to describe the state of the related art and the contents of the present invention more clearly.

### DISCLOSURE

### Technical Problem

The present inventors have made intensive studies to develop an excellent liquid formulation which improves the stability of plasma protein having a high risk of being contaminated and denatured immediately after separation and purification from blood, and is capable of maintaining the physical properties, biological activity and pharmacological effect of the plasma protein for a long period of time, especially even during lyophilization. As a result, the present inventors have found that, when a formulation comprises 0.2 mg/ml to 1.2 mg/ml of plasma protein as a therapeutic active ingredient, along with 40 mM to 200 mM of amino acid stabilizer, specifically, arginine, the formulation exhibits remarkably outstanding stability in terms of various indicators, including colloidal stability, refrigeration stability, inhibition of protein unfolding, blocking of protein aggregation, and cake appearance after lyophilization, thereby completing the present invention.

Accordingly, it is an object of the present invention to provide a pharmaceutical formulation for a plasma protein.

It is another object of the present invention to provide a composition for preventing or treating thrombotic disease.

Other objects and advantages of the present invention will become more apparent from the following detailed description of the invention, the appended claims and the accompanying drawings.

### Technical Solution

In one aspect of the present invention, there is provided a pharmaceutical formulation comprising 0.2 mg/ml to 1.2 mg/ml of plasma protein and 40 mM to 200 mM of amino acid stabilizer.

The present inventors have made intensive studies to develop an excellent liquid formulation which improves the stability of plasma protein having a high risk of being contaminated and denatured immediately after separation and purification from blood, and is capable of maintaining the physical properties, biological activity and pharmacological effect of the plasma protein for a long period of time, especially even during lyophilization. As a result, the present inventors have found that, when a formulation comprises 0.2 mg/ml to 1.2 mg/ml of plasma protein as a therapeutic active ingredient, along with 40 mM to 200 mM of amino acid stabilizer, specifically, arginine, the formulation exhibits remarkably outstanding stability in terms of various indicators, including colloidal stability, refrigeration stability, inhibition of protein unfolding, blocking of protein aggregation, and cake appearance after lyophilization.

As used herein, the term "plasma protein" refers to water-soluble proteins present in human or animal blood plasma, including all protein components in blood other than those contained in white blood cells and red blood cells. Plasma proteins account for about 8% of total plasma and are responsible for hemostasis (prothrombin and fibrinogen), hormone transport (serum albumin, lipoprotein, etc.), and immune action (immunoglobulin and complement proteins). Plasma proteins can be obtained by various fractionation and purification methods known in the art, but problems of chemical and physical instabilities due to long-term storage and environmental changes must be overcome. Physical instabilities involve modifications that do not lead to covalent change in proteins, namely adsorption, aggregation and precipitation, and chemical instabilities involve modifications such as deamidation, racemization, hydrolysis, oxidation, beta-elimination, and disulfide exchange. These instabilities lead to the alteration of intrinsic biological activity and reduction of pharmacological effects.

As used herein, the term "stabilizer" refers to any additive that is added to a formulation to increase the stability of an active ingredient and to prevent the active ingredient from being arbitrarily denatured, oxidized, aggregated, crystallized, or modified into a related substance, thereby preventing the pharmacological activity of the active ingredient from being lost or reduced. The stabilizer is not particularly limited as long as it is pharmaceutically acceptable. The term "amino acid stabilizer" refers to a stabilizer that induces the above-described stabilizing effect by adding an amino acid as a main component or an auxiliary component to a formulation.

According to a specific embodiment of the present invention, the amino acid is at least one selected from the group consisting of arginine (Arg), proline (Pro), and pharmaceutically acceptable salts thereof.

As used herein, the term "pharmaceutically acceptable salt" includes salts derived from pharmaceutically acceptable inorganic acids, organic acids, or bases. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, trifluroacetic acid, citric acid, methanesulfonic acid, formic acid, benzoic acid, malonic acid, naphthalene-2-sulfonic acid, benzenesulfonic acid, and the like. Examples of salts derived from suitable bases include salts of alkali metals such as sodium, alkaline earth metals such as magnesium, ammonium, and the like.

According to a specific embodiment of the present invention, the formulation of the present invention contains 0.25 mg/ml to 1.0 mg/ml plasma protein, specifically 0.3 mg/ml to 0.7 mg/ml plasma protein, more specifically 0.3 mg/ml to 0.4 mg/ml plasma protein, most specifically about 0.36 mg/ml plasma protein.

According to a specific embodiment of the present invention, the formulation of the present invention contains 60 mM to 180 mM amino acid stabilizer, specifically 60 mM to 160 mM amino acid stabilizer, more specifically 60 mM to 140 mM amino acid stabilizer, still more specifically 80 mM to 135 mM amino acid stabilizer, still more specifically 100 mM to 130 mM amino acid stabilizer, most specifically about 120 mM amino acid stabilizer.

According to a specific embodiment of the present invention, the formulation further contains a sugar stabilizer in an amount of 0 to 1.5 w/v% based on the total volume of the formulation.

More specifically, the formulation further comprises the sugar stabilizer in an amount of 0.2 to 1.5 w/v%, still more specifically 0.3 to 1.5 w/v%, still more specifically 0.5 to 1.5 w/v%, still more specifically 0.7 to 1.3 w/v%, still more specifically 0.9 to 1.1 w/v%, most specifically about 1 w/v%.

According to a specific embodiment of the present invention, the sugar is at least one selected from the group consisting of sucrose, trehalose, and pharmaceutically acceptable salts thereof.

According to a specific embodiment of the present invention, the formulation further comprises 100 mM to 400 mM of inorganic salt.

As used herein, the term "inorganic salt" refers to a salt derived from an inorganic substance containing no C-H bond, which is a salt consisting of an ionic combination of cations and anions in an aqueous solution. Examples of inorganic salts that may be used in the present invention include, but are not limited to NaCl, CaCl₂, KCl, MgCl₂, and combinations thereof. Specifically, the inorganic salt of the present invention is a mixture of NaCl and CaCl₂.

According to a specific embodiment of the present invention, NaCl in the inorganic salt may be contained at a concentration of 140 mM to 370 mM, specifically 180 mM to 340 mM, more specifically 220 mM to 310 mM, still more specifically 260 to 300 mM, most specifically about 280 mM.

According to a specific embodiment of the present invention, CaCl₂ in the inorganic salt may be contained at a concentration of 2 mM to 6 mM, specifically 3 mM to 5 mM, most specifically about 4 mM.

According to a specific embodiment of the present invention, the formulation of the present invention contains 10 mM to 30 mM histidine, more specifically 15 mM to 25 mM histidine, most specifically about 20 mM histidine.

According to a specific embodiment of the present invention, the formulation further comprises a nonionic surfactant in an amount of 0.01 to 0.1 v/v% based on the total volume of the formulation.

As used herein, the term "surfactant" refers to a soluble compound that is used to increase the water solubility of a hydrophobic material or to increase the compatibility of a plurality of materials having different hydrophobicity. The term "nonionic surfactant" refers to a surfactant that dissolves without dissociation even in an aqueous solution because the entire molecular structure thereof does not contain ionized functional groups or atomic groups.

According to a specific embodiment of the present invention, the nonionic surfactant that may be used in the present invention is at least one selected from the group consisting of polysorbate 80, polysorbate 60 and polysorbate 40, and is more specifically polysorbate 80.

According to a specific embodiment of the present invention, the formulation of the present invention comprises 0.03 to 0.08 v/v% of the nonionic surfactant, most specifically about 0.05 v/v% of the nonionic surfactant.

According to a specific embodiment of the present invention, the plasma protein to which the formulation of the present invention is applied is ADAMTS13 (a disintegrin and metalloproteinase with a thrombospondin type 1 motif, member 13) protein, a variant thereof, or a functional fragment thereof.

As used herein, the term "functional fragment" refers to a fragment resulting from deletion of one or more amino acid residues from a full-length protein, which is a full-length protein analogue that retains the biological activity and function of the full-length protein.

According to the present invention, SEQ ID NO: 1 is the amino acid sequence of ADAMTS13 protein consisting of 1,427 amino acids. Accordingly, the ADAMTS13 variant protein or a functional fragment thereof according to the present invention may be an ADAMTS13 variant in which amino acid mutation has been introduced in the full-length (1,427 a.a.) ADAMTS13 protein or a fragment thereof comprising including a 75-685 a.a. region. The fragment comprising the 75-685 a.a. region may be, for example, a 1-685 a.a. (685 a.a) or 75-685 a.a. (611 a.a) region.

SEQ ID NO: 1, which is an amino acid sequence of the ADAMTS13 protein and essentially comprises the functional fragment of ADAMTS13 protein, also includes an amino acid sequence showing substantial identity to the above sequence. As used herein, the term "substantial identity" refers to an amino acid sequence showing at least 70% homology, specifically at least 80% homology, more specifically at least 90% homology, most specifically at least 95% homology, when aligning the amino acid sequence with any other sequence so as to correspond as much as possible and analyzing the aligned sequence using an algorithm commonly used in the art.

According to a specific embodiment of the present invention, the variant of the ADAMTS13 protein comprises substitution of at least one amino acid residue selected from the group consisting of residues at positions 85, 93, 126, 135, 278, 282, 308, 314, 317, 334, 364, 376, 413, 427, 452, 465, 567, 578, 585, 589, 607, 608, 609, 612, 618, 624, 630, 635, 643, 650, 651, 654, 655, 656, 658, 664 and 672 of SEQ ID NO: 1.

The present inventors have identified core regions recognized by anti-ADAMTS13 autoantibodies, and have found that, when substitution of some amino acids in these regions is made, binding of these regions to the autoantibodies may be blocked and vWF degradation activity and thrombosis inhibitory activity thereof may be maintained. Therefore, an ADAMTS13 protein with mutations at above positions may be used as an effective therapeutic composition for various diseases caused by excessive thrombus formation, including thrombotic thrombocytopenic purpura (TTP).

As used herein, the term "autoantibody" refers to an antibody that is produced by a subject's own immune system and recognizes and targets an subject's own protein. The autoantibody is an immunoglobulin protein that comprises at least one variable domain binding to an epitope of an antigen and specifically recognizes the antigen. The presence of autoantibodies causes degradation or loss of the intrinsic function or biological activity of proteins specifically recognized by the autoantibodies, and thus causes various diseases.

According to a specific embodiment of the present invention, the variant of the ADAMTS13 protein is selected from the group consisting of variant proteins comprising substitution of an amino acid residue at:
positions 85 and 317; position 612; two or more of positions 282, 465 and 672; position 635; positions 452 and 612; two or more of positions 278, 334 and 427; position 618; position 135; two or more of positions 126, 567 and 651; position 413; position 334; position 314; two or more of positions 93, 364 and 376; position 308; position 656; position 607; positions 612 and 624; position 589; positions 650 and 656; position 643; positions 585 and 658; two or more of positions 630, 654 and 664; four or more of positions 589, 608, 609, 624 and 655; position 578; position 585; positions 314 and 635; and positions 314 and 612.

More specifically, the substitution of the amino acid residue is at least one selected from the group consisting of substitution with Phe at position 85, substitution with Val at position 93, substitution with Met at position 126, substitution with Ile at position 135, substitution with Ile at position 278, substitution with Ala at position 282, substitution with Lys at position 308, substitution with Thr at position 314, substitution with His at position 317, substitution with Thr or Val at position 334, substitution with Arg at position 364, substitution with Asp at position 376, substitution with Asp at position 413, substitution with Asn at position 427, substitution with Ile at position 452, substitution with Asp at position 465, substitution with Ser at position 567, substitution with Leu at position 578, substitution with Asn or Met at position 585, substitution with Gln at position 589, substitution with Arg at position 607, substitution with Met at position 608, substitution with Leu at position 609, substitution with Phe or Tyr at position 612, substitution with Ser at position 618, substitution with Asp or Cys at position 624, substitution with Leu at position 630, substitution with Val at position 635, substitution with Phe at position 643, substitution with His at position 650, substitution with Asp at position 651, substitution with Gly at position 654, substitution with Val at position 655, substitution with Arg or His at position 656, substitution with His at position 658, substitution with Asn at position 664, and substitution with Val at position 672.

According to a specific embodiment of the present invention, the plasma protein of the present invention is conjugated with an Fc region derived from IgG4 immunoglobulin.

The present inventors have found that, when the Fc region derived from IgG4 immunoglobulin is conjugated to the ADAMTS13 variant protein of the present invention, the *in vivo* stability of the variant protein is greatly increased while maintaining the intrinsic vWF cleavage activity and neutralizing antibody evasion activity thereof, and in particular, structural instability appearing in open-form fragments from which a portion of the C-terminus of ADAMTS 13 has been removed is significantly reduced.

According to a more specific embodiment of the present invention, the Fc region comprises substitution of at least one amino acid residue selected from the group consisting of residues at positions 22, 24 and 26 of SEQ ID NO: 2. More specifically, the substitution of the amino acid residue is at least one selected from the group consisting of substitution with Tyr at position 22, substitution with Thr at position 24, and substitution with Glu at position 26.

According to the present invention, SEQ ID NO: 2 is the amino acid sequence of an Fc region (217 a.a.) derived from IgG4 immunoglobulin. The present inventors have found that, when the IgG4 immunoglobulin-derived Fc region [IgG4 (YTE)] in which residues at positions 22, 24 and 26 are substituted with Tyr, Thr and Glu, respectively, is fused to the above-described ADAMTS13 variant protein or a functional fragment thereof, the blood half-life of the protein may be maximized and the physiological activity thereof after administration may be sustained for a long time.

According to a specific embodiment of the present invention, a hinge region derived from IgG1 immunoglobulin is further comprised between the plasma protein and the Fc region derived from IgG4 immunoglobulin.

According to the present invention, the hinge region derived from IgG1 immunoglobulin may be represented by SEQ ID NO: 3 (15 a.a.).

In another aspect of this invention, there is provided a composition for preventing or treating thrombotic disease, the composition comprising, as an active ingredient, the above-described pharmaceutical formulation of the present invention, which contains ADAMTS13 protein as a plasma protein, a variant thereof, or a functional fragment thereof.

As used herein, the term "thrombotic disease" refers to a systemic disease in which blood flow is reduced or blocked due to blood clots generated by platelet aggregation in the microcirculatory system of blood vessels, which causes ischemic damage to organs such as the kidney, heart, and brain.

If the activity of the ADAMTS13 enzyme is inhibited by neutralizing antibodies and fails to properly degrade Willebrand factor (vWF), excessive platelet aggregation and overproduction of thrombi occur. Therefore, the ADAMTS13 variant protein of the present invention, which evades neutralizing antibodies with high efficiency while maintaining or improving its vWF degradation activity, may be as an effective preventive or therapeutic composition for various thrombotic diseases.

As used herein, the term "prevention" means suppressing the occurrence of a disorder or disease in a subject who has not been diagnosed with the disorder or disease, but is likely to suffer from the disorder or disease.

As used herein, the term "treatment" refers to (a) inhibiting the development of a disorder, disease or condition; (b) alleviating a disorder, disease or condition; or (c) eliminating a disorder, disease or condition. When the composition of the present invention is administered to a subject, it acts to specifically recognize and degrade vWF regardless of the presence or absence of neutralizing antibodies, thereby blocking the formation of blood clots, thereby inhibiting the progress of thrombotic diseases, or eliminating or alleviating thrombotic diseases. Therefore, the composition of the present invention may be a composition for treating these diseases by itself, or may be administered as a therapeutic adjuvant together with other pharmacological ingredients for the treatment of the above diseases. Accordingly, the term "treatment" or "therapeutic agent" as used herein includes the meaning of "therapeutic aid" or "therapeutic adjuvant".

As used herein, the term "administration" or "administer" refers to directly administering a therapeutically effective amount of the composition of the present invention to a subject so that the same amount is formed in the subject's body.

As used herein, the term "therapeutically effective amount" means a content of the pharmaceutical composition that is sufficient to provide the therapeutic or prophylactic effect of a pharmacological component contained in the composition to a subject to which the composition is to be administered, and accordingly, is meant to include a "prophylactically effective amount".

As used herein, the term "subject" includes, without limitation, a human, mouse, rat, guinea pig, dog, cat, horse, cow, pig, monkey, chimpanzee, baboon or rhesus monkey. Specifically, the subject of the present invention is a human.

According to a specific embodiment of the present invention, the thrombotic disease is thrombotic microangiopathy (TMA). More specifically, the thrombotic microangiopathy is selected from the group consisting of thrombocytopenic purpura (TTP), hemolytic uremic syndrome (HUS), HELLP (Hemolysis, Elevated Liver enzymes, Low Platelet count), preeclampsia, and sickle cell disease. Still more specifically, the thrombotic microangiopathy is thrombocytopenic purpura or sickle cell disease. Most specifically, the thrombotic microangiopathy is thrombocytopenic purpura.

In another aspect of this invention, there is provided a method for preventing or treating thrombotic disease, the method comprising administering to a subject the above-described pharmaceutical formulation of the present invention, which comprises ADAMTS13 protein as plasma protein, a variant thereof, or a functional fragment thereof.

As the plasma protein used in the present invention, the components for formulating the same, and thrombotic diseases that may be prevented or treated therewith are as already described above, they are omitted herein to avoid excessive overlaps.

### Advantageous Effects

The features and advantages of the present invention are summarized as follows:
(a) The present invention provides a pharmaceutical formulation of a plasma protein, specifically ADAMTS-13 protein, and a composition for preventing or treating thrombotic disease comprising the same.
(b) The pharmaceutical formulation of the present invention significantly improves the stability of plasma protein whose pharmacological activity and quality deteriorates during long term storage due to its high risk of being contaminated and denatured immediately after separation and purification from blood, and in particular, is capable of maintaining the colloidal stability, refrigeration stability, purity, and inhibition of aggregation of the plasma protein at high levels, and allows the cake appearance after lyophilization to be maintained well for a long period of time. Accordingly, the pharmaceutical formulation of the present invention may be advantageously used to maintain and store ADAMTS-13 protein, which is an important therapeutic resource for use for various thrombotic diseases, for a long period of time without loss of the therapeutically effective amount of the protein.

### Brief Description of Drawings

FIG. 1 shows the results of measuring B₂₂ values to evaluate the colloidal stability of formulations containing five amino acid stabilizers, respectively.
FIG. 2 shows the results of performing size exclusion liquid chromatography (SE-HPLC) to evaluate the recovery of monomer at room temperature after the addition of each of seven stabilizers (six amino acids and sucrose).
FIG. 3 shows the results of evaluating refrigeration stability by SE-HPLC depending on the addition of 20 mM arginine compared to a control.
FIG. 4 shows the results of SE-HPLC performed to evaluate purity and stability depending on the concentration of protein (ADAMTS-13), which is a pharmacological ingredient, and on the concentration of arginine.
FIG. 5 shows cake appearances (FIG. 5a) at varying NaCl concentrations and the results of evaluating purity at varying NaCl concentrations by SE-HPLC (FIG. 5b).
FIG. 6 shows the results of performing SE-HPLC depending on the NaCl concentration in liquid formulations in the presence of 20 mM arginine.
FIG. 7 shows cake appearances (FIG. 7a) and SE-HPLC results (FIG. 7b) depending on the concentration of sucrose and the addition or non-addition of trehalose in the presence of 120 mM NaCl.
FIG. 8 shows cake appearances (FIG. 8a) and SE-HPLC results (FIG. 8b) depending on the mixing ratio between sucrose and mannitol or glycine, which is a bulking agent.
FIG.9 compares cake appearances depending on the concentration of glycine in the presence of 120 mM NaCl.
FIG.10 compares cake appearances depending on the concentration of arginine in the presence of 120 mM NaCl.
FIG. 11 shows cake appearances (FIG. 11a) and SE-HPLC results (FIG. 11b) depending on the mixing ratio of NaCl to sucrose in the absence of arginine.
FIG. 12 shows cake appearances (FIG. 12a) and SE-HPLC results (FIG. 12b) depending on the mixing ratio of NaCl to sucrose in the absence of 120 mM arginine.
FIG. 13 shows the results of measuring the change in purity after 1 month at 40°C depending on the mixing ratio of NaCl to sucrose.
FIG.14 shows the results of evaluating the effect of preventing agitation-induced aggregation depending on the concentration of polysorbate 80 by observing appearances (FIG. 14a), measuring turbidity (FIG. 14b), and performing SE-HPLC (FIG. 14c).
FIG. 15 shows the results of evaluating the liquid phase stability depending on the concentration of polysorbate 80 by SE-HPLC.
FIG. 16 shows the results of evaluating the formulation quality after lyophilization depending on the concentration of polysorbate 80 by performing SE-HPLC (FIG. 16a) and determining whether or not higher-order aggregates are generated (FIG. 16b).
FIG. 17 shows the results of measuring thermal unfolding (Tₘ) (FIG. 17a) and evaluating thermal aggregation (T_{agg}) (FIG. 17b), depending on pH.
FIG. 18 shows lyophilized cake appearances (FIG. 18a) and SE-HPLC analysis results (FIG. 18b) depending on the concentration of CaCl₂.
FIG. 19 shows the results of evaluating appearances depending on the NaCl and sucrose concentrations (FIG. 19a) and shows cake appearances at each concentration (FIG. 19b).
FIG. 20 shows cake appearances depending on the NaCl and sucrose concentrations.
FIG. 21 is a graph showing the results of evaluating colloidal stability depending on the arginine concentration.
FIG. 22 shows cake appearances (FIG. 22a) and purity (FIG. 22b) depending on the arginine concentration.
FIG. 23 shows the physical properties of a liquid formulation finally selected in the present invention, and specifically, shows the time-dependent change in purity (FIG. 23a), the time-dependent change in potency (FIG. 23b), the time-dependent change in protein concentration (FIG. 23c), and the time-dependent change in turbidity (FIG. 23d).
FIG. 24 shows long-term storage stability depending on the NaCl and sucrose concentrations, and specifically, shows the changes in purity (FIG. 24a) and potency (FIG. 24b) at the initial stage of storage and after 6 months of storage.

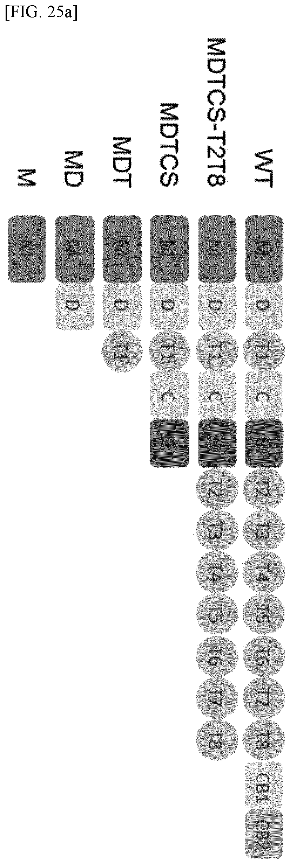

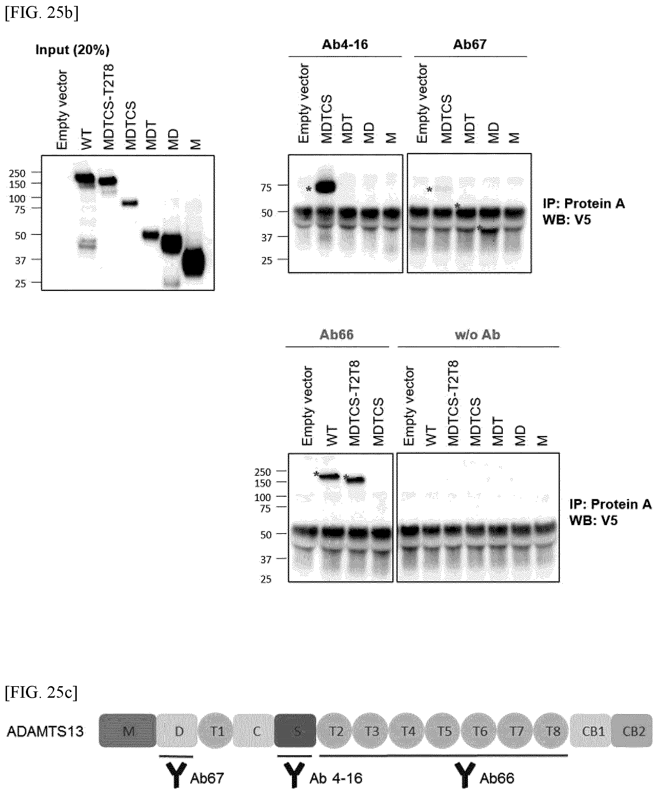

FIG. 25 shows the results of identifying the binding site of each domain of ADAMTS13 that binds to neutralizing antibodies. Specifically, FIG. 25a schematically shows six types of ADAMTS13 fragments (or wild-type full-length ADAMTS13) having combinations of various domains, constructed to confirm the expression rate or neutralizing antibody-binding site of ADAMTS13. FIG. 25b shows the results of performing Western blotting with anti-V5 antibody after performing immunoprecipitation with a mixture of protein A-Sepharose and each neutralizing antibody using proteins expressed from each ADAMTS13 domain. Input shows the level of protein expression from each domain. FIG. 25c schematically shows the neutralizing antibody binding sites of ADAMTS13, and shows that the Ab 4-16 antibody specifically binds to domain S, the Ab 67 antibody to domain D, and the Ab 66 antibody to domains T2 to T8 domain.
FIG. 26 shows the results of screening for variants that evade anti-ADAMTS13 antibodies or have better activity than wild-type ADAMTS13. The Ab 4-16 antibody (FIG. 26a) and Ab 67 antibody (FIG. 26b) escaping rates and ADAMTS13 activities of wild-type clones and ADAMTS13 variants were measured. The relative activity was calculated by determining the specific activity of the wild-type clone and the variant and substituting the determined values into the following equation: Relative activity (%) = specific activity of variant/specific activity of wild-type ADAMTS13 X 100

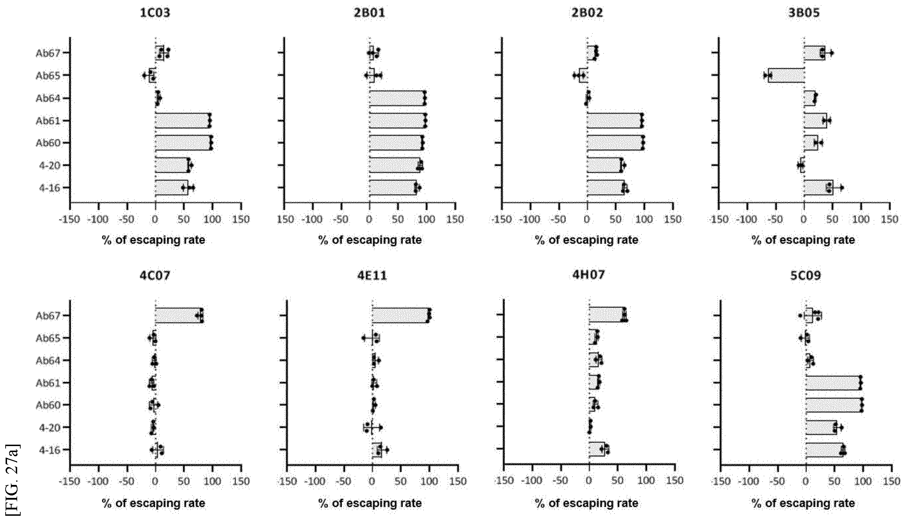

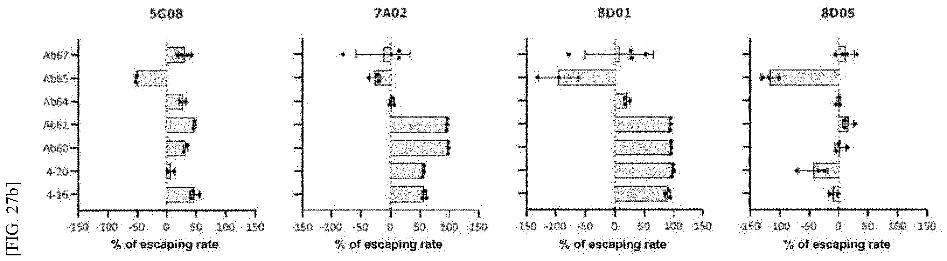

FIGS. 27a and 27b show the results of measuring the single neutralizing antibody escaping rates of 12 full-length ADAMTS13 variants. The antibody escaping rates of 12 full-length ADAMTS13 variants for 7 neutralizing antibodies (Ab4-16, Ab4-20, Ab60, Ab61, Ab64, Ab65, and Ab67) were measured. The neutralizing antibody escaping rate was calculated by determining the relative binding level in comparison with that of the WT ADAMTS13 to determine the relative escaping rate, and then substituting the relative escaping rate into the following equation: Escaping rate (%) = (1- binding affinity of variant/binding affinity of WT ADAMTS13) x 100

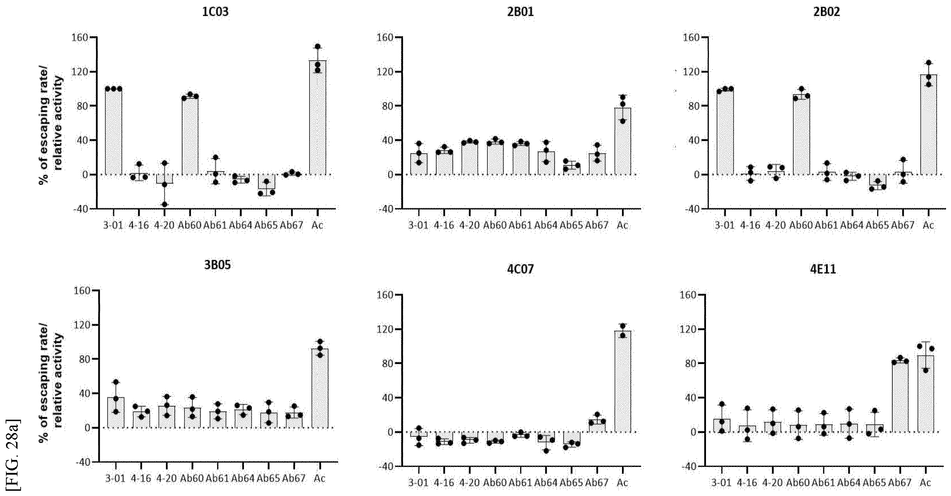

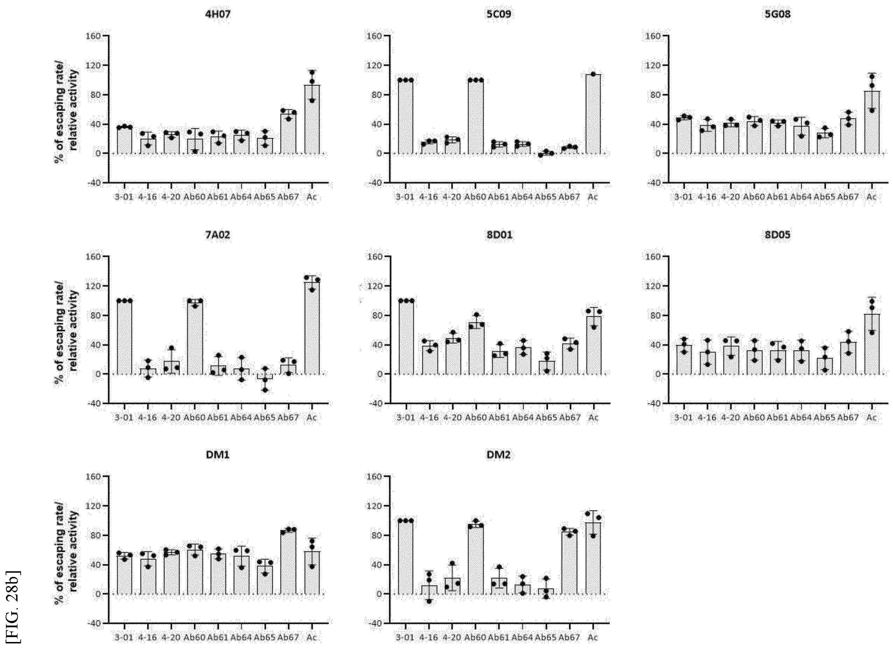

FIGS. 28a and 28b show the results of measuring single neutralizing antibody escaping rates under the culture medium condition in which each Fc-conjugated MDTCS fragment variant has been expressed. An MDTCS fragment was obtained from each of twelve selected variants and Fc was conjugated thereto, thus obtaining 12 MDTCS fragment variants. In addition, two variants (DM1 and DM2) having two amino acid mutations were obtained by combining the selected variant amino acid residues. Using the culture medium in which each of the 14 variants (including the 12 MDTCS fragment variants and the two variants) has been expressed, the neutralizing antibody escaping rates of the variants for 8 single neutralizing antibodies (Ab3-01, Ab4-16, Ab4-20, Ab60, Ab61, Ab64, Ab65, and Ab67) and the relative ADAMTS13 activities of the variants were measured. Escaping rate (%) = (1- binding affinity to variant/binding affinity) x 100; relative activity (%) = specific activity of variant/specific activity of WT ADAMTS13 x 100

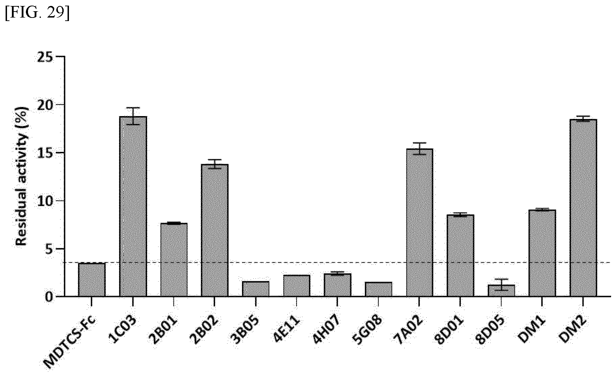

FIG. 29 shows the results of evaluating the ability of each Fc-conjugated MDTCS fragment variant to evade mixed neutralizing antibodies under the culture medium condition in which each Fc-conjugated MDTCS fragment variant has been expressed. Nine neutralizing antibodies (Ab3-01, Ab4-16, Ab4-20, Ab60, Ab61, Ab64, Ab65, Ab66, and Ab67) mixed together in equal proportions were mixed and incubated with 4 nM culture medium in which each of the control MDTCS-Fc and 12 candidate variants has been expressed, and then the residual activity of each candidate variant was calculated using the following equation: Residual activity (%) = A ÷ B x 100 (A: activity under condition treated with mixed neutralizing antibodies, and B: activity under condition not treated with neutralizing antibodies).

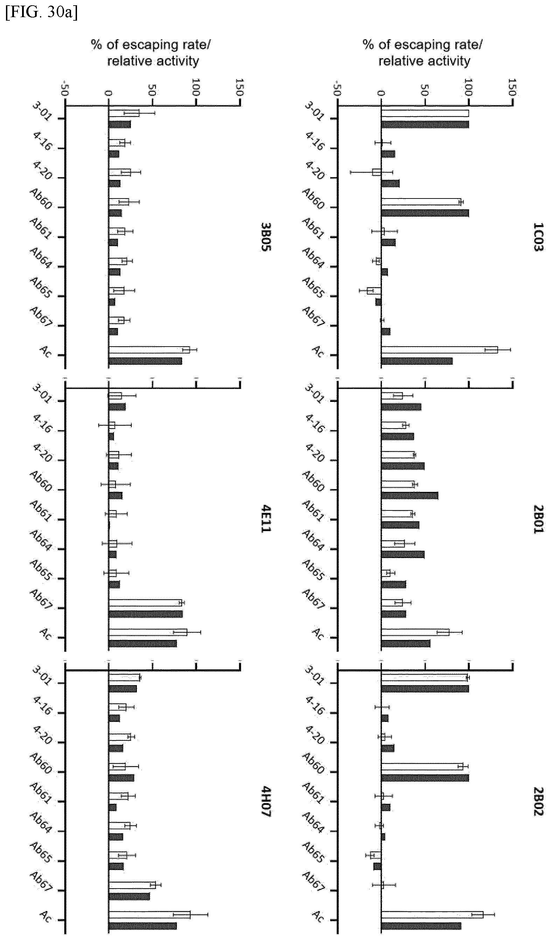

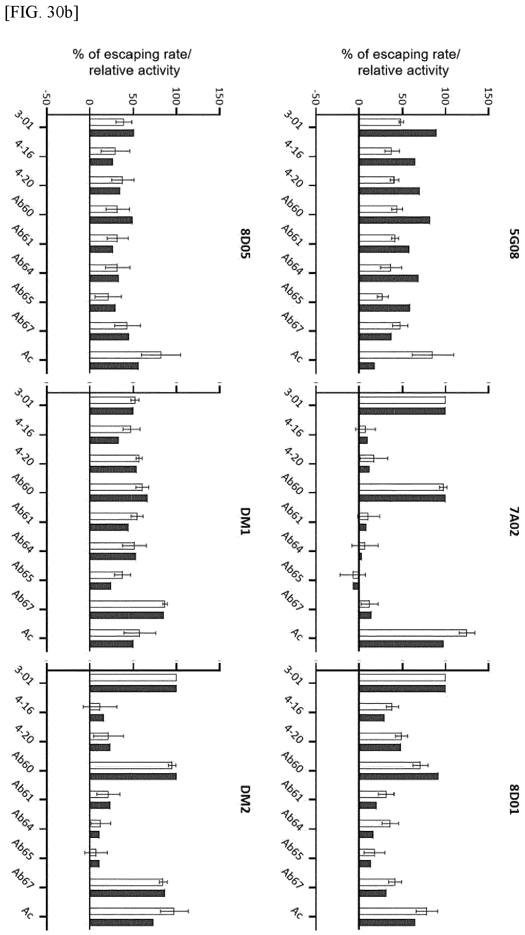

FIGS. 30a and 30b show the results of evaluating single neutralizing antibody escaping rate under culture medium and purified solution conditions in which each Fc-conjugated MDTCS fragment variant has been expressed. Using a culture medium in which each of 12 variants (1C03, 2B01, 2B02, 3B05, 4E11, 4H07, 5G08, 7A02, 8D01, 8D05, DM1, and DM2) has been expressed or a purified solution obtained through purification using the Phytip system, the neutralizing antibody escaping rates of each variant for 8 single neutralizing antibodies (Ab3-01, Ab4-16, Ab4-20, Ab60, Ab61, Ab64, Ab65, and Ab67) and the relative activities of each variant were measured (FIGS. 30a and 30b). The concentration in the corresponding purified solution was determined by Fc ELISA. Escaping rate (%) = (1- binding affinity to variant/binding affinity to MDTCS-Fc) x 100; relative activity (%) = specific activity of variant /specific activity of MDTCS-Fc x 100. The blue bar represents the median value of the purified variant protein, and the gray bar represents the median value of the expressed variant protein.

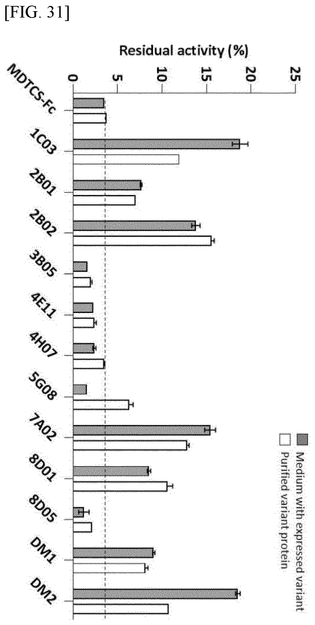

FIG. 31 shows the results of measuring mixed neutralizing antibody escaping rates under culture medium and purified solution conditions in which each Fc-conjugated MDTCS fragment variant has been expressed. Eight neutralizing antibodies (Ab3-01, Ab4-16, Ab4-20, Ab60, Ab61, Ab64, Ab65, and Ab67) mixed together in equal proportions were mixed and incubated with 4 nM culture medium or purified solution in which each of the control MDTCS-Fc and 12 candidate variants has been expressed, and then the residual activity of each variant was measured. The residual activity was calculated using the following equation. Residual activity (%) = A ÷ B x 100 (A: activity under condition treated with mixed neutralizing antibodies, and B: activity under condition not treated with neutralizing antibodies).
FIG. 32 is a graph showing the pharmacokinetic results of Fc-conjugated MDTCS fragment variants. Each of MDTCS, Fc (IgG1-YTE)-conjugated MDTCS, and four final candidate variant (1C03, 5C09, 7A02, and DM2) fragment proteins to which IgG1-YTE has been conjugated was administered to mice via the tail vein, and then plasma was collected over time. Each substance was administered to reach 160 IU/kg based on the specific activity thereof, and the activity of the substance remaining in plasma obtained at each time point was measured by activity assay.

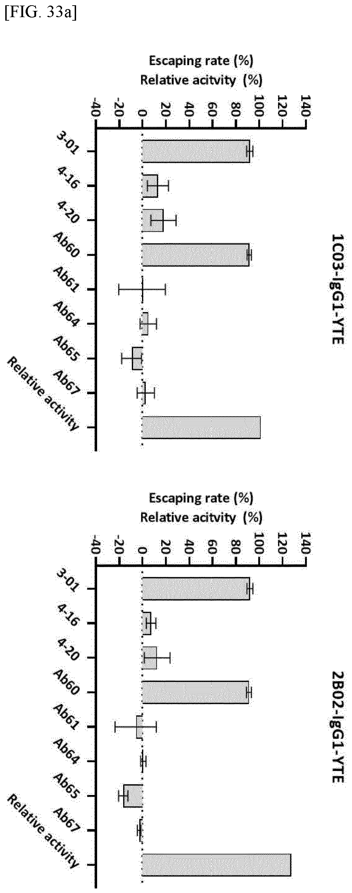

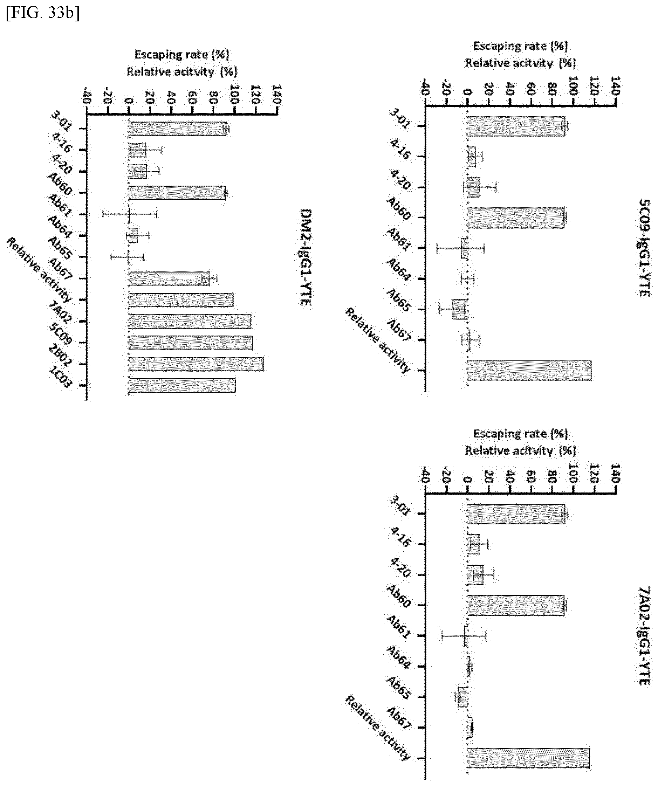

FIG. 33 shows the results of evaluating single neutralizing antibody escaping rates under the culture medium condition in which each IgG1-YTE-conjugated MDTCS fragment variant has been expressed. An MDTCS fragment was obtained from five selected variants and IgG1-YTE was conjugated thereto. Using the culture medium in which each IgG1-YTE-conjugated variant has been expressed, the neutralizing antibody escaping rates of each IgG1-YTE-conjugated variant for 8 single neutralizing antibodies (Ab3-01, Ab4-16, Ab4-20, Ab60, Ab61, Ab64, Ab65, and Ab67) and the relative activities of each IgG1-YTE-conjugated variant were measured (FIGS. 33a and 33b). Escaping rate (%)=(1-binding affinity to variant/binding affinity to MDTCS-IgG1-YTE) x 100; relative activity (%) = specific activity of variant/specific activity of MDTCS-IgG1-YTE x 100.

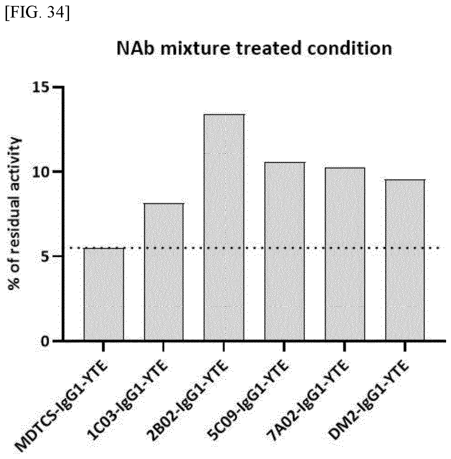

FIG. 34 shows the results of evaluating the ability to evade mixed neutralizing antibodies under the culture medium condition in which each IgG1-YTE-conjugated MDTCS fragment variant has been expressed. Nine neutralizing antibodies (Ab3-01, Ab4-16, Ab4-20, Ab60, Ab61, Ab64, Ab65, Ab66, and Ab67) mixed together in equal proportions were mixed and incubated with 4 nM culture medium in which each of the control MDTCS-IgG1-YTE and the five candidate variants has been expressed, and then the residual activity of each candidate variant was calculated using the following equation: residual activity (%)= A ÷ B x 100 (A: activity under condition treated with mixed neutralizing antibodies, and B: activity under condition not treated with neutralizing antibodies).

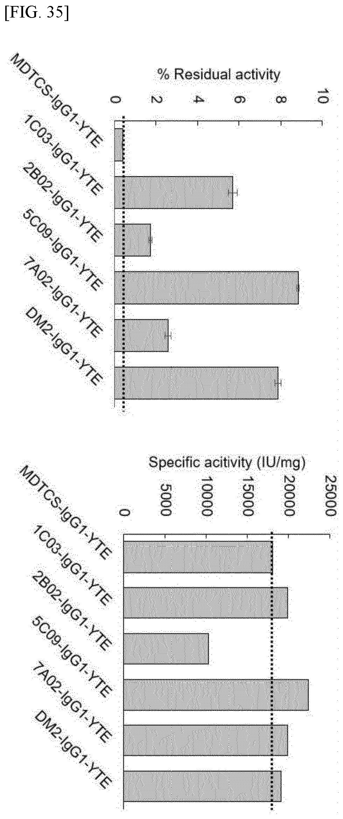

FIG. 35 shows the results of evaluating the ability of each IgG1-YTE-conjugated MDTCS fragment variant to evade mixed neutralizing antibodies under the purified solution condition. Nine neutralizing antibodies (Ab3-01, Ab4-16, Ab4-20, Ab60, Ab61, Ab64, Ab65, Ab66, and Ab67) mixed together in equal proportions were mixed and incubated with 4 nM purified solution of each of the control MDTCS-IgG1-YTE and five candidate variants, and then the residual activity of each candidate variant was calculated using the following equation: residual activity (%) = A ÷ B x 100 (A: activity under condition treated with mixed neutralizing antibodies, and B: activity under condition not treated with neutralizing antibodies).
FIG. 36 schematically shows a procedure for testing neutralizing antibody escaping rate using an aTTP-mimic mouse model (FIG. 36a), and shows the dose-dependent residual ADAMTS 13 activity of each variant candidate (FIG. 36b).
FIG. 37 shows a schematic diagram of a procedure for testing the dose-dependent residual ADAMTS13 activity of DM2-IgG1-YTE, which exhibited the highest neutralizing antibody escaping rate, and clinical symptoms using a TTP-mimic mouse model (FIG. 37a), the results of observing improved platelet and LDH levels and ADAMTS13 activity (FIG. 37b), and the results of observing clinical symptoms (FIG. 37c).
FIG. 38 shows a schematic diagram of a procedure for testing whether administration of DM2-IgG1-YTE to a cTTP mouse model alleviates hematological and clinical symptoms and testing the degree of restoration of human ADAMTS13 activity (FIG. 38a), and the results of observing improved platelet and LDH levels and the results of observing the restoration of ADAMTS13 activity (FIG. 38b).

### Mode for Invention

Hereinafter, the present invention will be described in more detail with reference to examples. These examples are only for explaining the present invention in more detail, and it will be apparent to those skilled in the art that the scope of the present invention according to the subject matter of the present invention is not limited by these examples.

### Examples

### Example 1: Selection of components for formulation of ADAMTS13 protein

Each component and content of a liquid formulation of the ADAMTS13 protein described in Example 3 below were determined through the following process.

### Lyophilization process

After completion of formulation, 1.0 ml of each sample solution was dispensed into each glass vial (3 ml) which was then partially stoppered with a rubber stopper and loaded onto a shelf of a freeze dryer (Lyostar 3, SP Scientific). Thereafter, lyophilization was performed under the conditions shown in Table 1 below, and the prepared lyophilized formulation was capped with an aluminum cap after completion of lyophilization.

**[Table 1]**

| Step | Operation | Rate/hold | Shelf temperature | Time (min) | Vacuum level (mTorr) |
|---|---|---|---|---|---|
| 1 | Loading | Hold | 5°C | 20 | N/A |
| 2 | Lyophilization | Rate | -55°C | 200 | N/A |
| 3 | Lyophilization | Hold | -55°C | 300 | N/A |
| 4 | First warming | Rate | -25°C | 100 | 60 |
| 5 | First drying | Hold | -25°C | 2000 | 60 |
| 7 | Second warming | Rate | 25°C | 167 | 50 |
| 8 | Second drying | Hold | 25°C | 600 | 50 |

The stability of the prepared lyophilized formulation was analyzed after reconstitution with 1.0 ml of distilled water.

### Size exclusion liquid chromatography (SE-HPLC)

For size exclusion liquid chromatography, a sample was diluted with a mobile phase (1 x PBS, Lonza) to 1.0 mg/ml (a sample at 1.0 mg/mml or less was undiluted) and then sterile-filtered. 200 µL of the filtered sample was inserted into a vial insert which was then inserted into a screw top vial. Next, the mobile phase was connected to the pump, and then an analytical column (TSKgel G3000SWXL, Tosoh) was mounted in the Waters e2695 & Waters 2489 system (Waters, Japan) while allowing the mobile phase to flow at a flow rate of 0.5 mL/min. The mobile phase was allowed to flow at a rate of 0.5 mL/min for more than 30 minutes to reach equilibrium until the detector signal was stabilized. When the temperature of the autosampler dropped to 4°C, the sample was inserted into the sampler. 30 µL of the sample was injected, and then the mobile phase was flowed for 35 minutes, and the detection peak at 280 nm was measured. Then, analysis was performed with Empower Pro software on a PC.

### Colloidal stability (B₂₂)

Since the degree of protein-protein interaction affects aggregation and solubility, colloidal stability is an important item that must be considered in the development of protein formulations. B₂₂ (second virial coefficient), a representative colloidal stability indicator, was used. In general, when the B₂₂ value is a large positive value, the repulsion force between proteins is strong and the probability of occurrence of aggregation is reduced.

To evaluate the colloidal stability of five stabilizers, 100 mM glycine, lysine, proline, alanine or arginine as an amino acid was added to a basic composition (pH 7.4) containing 1.2 mg/ml ADAMTS protein, 20 mM histidine, 4.0 mM CaCl₂, 120 mM NaCl, and 1.0% sucrose.

A liquid sample (B₂₂) of each of the formulated compositions was analyzed for colloidal stability using a UNCLE device.

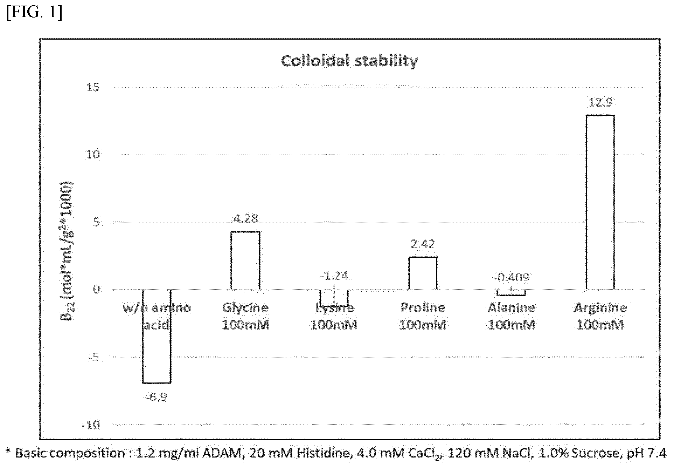

A higher positive B₂₂ value means that the protein is well dispersed without aggregation. Thus, as shown in FIG. 1, it was confirmed that the colloidal stability was higher in the order of arginine > glycine > proline > alanine > lysine > control.

### Turbidity analysis

Turbidity was analyzed using Lunatic (Unchained Labs). 2.0 µL of a sample was injected into a Lunatic plate (Unchained Labs) and the turbidity at 350 nm was measured. The final value was calculated by subtracting the turbidity of placebo buffer from the turbidity value of the sample.

### Thermal unfolding and thermal aggregation analysis

The degree of protein unfolding can be measured by detecting the emission wavelength of tryptophan exposed on the surface when protein is unfolded as the temperature increases. The UNCLE (Unchained Labs) system was used for differential scanning fluorimetry analysis based on such intrinsic fluorescence intensity. To analyze the thermal stability of proteins using the above system, Tₘ (thermal unfolding) and T_{agg} (thermal aggregation) were measured as follows:
8.8 µL of a sample was injected twice into the Uni sample loader (Unchained Labs), and then the temperature was increased from 25°C to 95°C at a rate of 1°C/min. The intensity of a wavelength (250-720 nm) emitted at an excitation wavelength of 266 nm was measured while increasing the temperature. For fluorescence data analysis, UNCLE analysis software (Unchained Labs) was used.

Through the above analysis, the temperature at the maximum value of the fluorescence emission peak was defined as Tₘ. The static light scattering of the protein at 266 nm (SLS266) was measured, and the temperature at which protein aggregation started (protein aggregation onset temperature) was defined as T_{agg}.

### SE-HPLC evaluation for seven stabilizers

100 mM arginine, serine, valine, threonine, proline or glycine as an amino acid stabilizer was added to a basic composition (pH 7.4) containing 0.9 mg/ml ADAMTS protein, 20 mM histidine, 2.0 mM CaCl₂ and 120 mM NaCl, or 1.0 w/v% of sucrose as a sugar stabilizer was added to the basic composition.

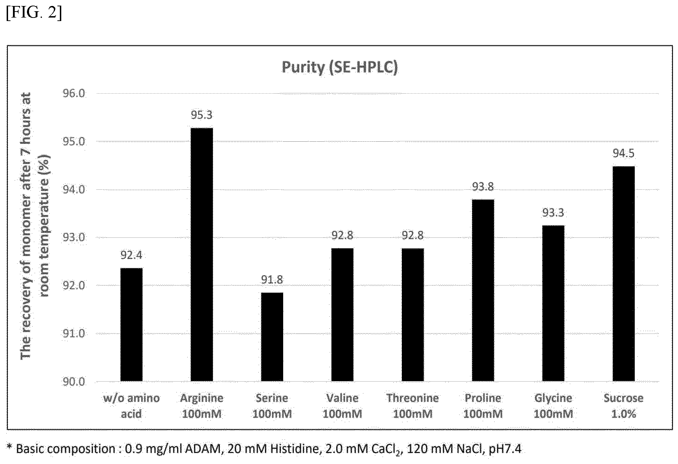

After completion of formulation, a liquid sample of each composition was stored at room temperature for 7 hours and then subjected to SE-HPLC analysis. As a result, as shown in FIG. 2, it was confirmed that arginine exhibited the best liquid phase stability among the seven stabilizers tested for comparison, and stability and purity were higher in the order of arginine > sucrose > proline > glycine > valine, threonine > control > serine.

### Evaluation of liquid phase stability depending on addition of arginine

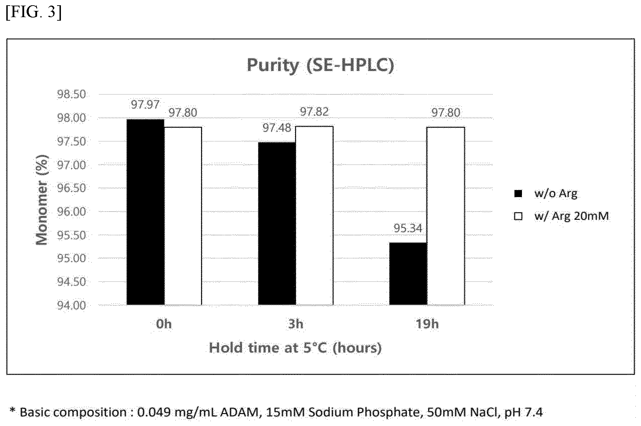

To evaluate liquid phase stability depending on whether or not 20 mM arginine was added to a composition (pH 7.4) containing 0.049 mg/mL ADAMTS protein, 15 mM sodium phosphate, and 50 mM NaCl, the formulated liquid sample was stored at room temperature for 3 hours and 19 hours and then analyzed by SE-HPLC at each analysis time point. As a result, as shown in FIG. 3, it was confirmed that the refrigeration stability at 5°C was significantly improved compared to the control when 20 mM arginine was added.

### Evaluation of liquid phase stability depending on target protein concentration and arginine concentration

### SE-HPLC analysis

To a basic composition (pH 7.4) containing 20 mM histidine, 4.0 mM CaCl₂ and 160 mM NaCl, ADAMTS protein was added at concentrations of 0.2, 0.6 and 1.0 mg/ml, and arginine was added at concentrations of 20, 80 and 140 mM, thus preparing liquid samples. The stability of each liquid sample was evaluated.

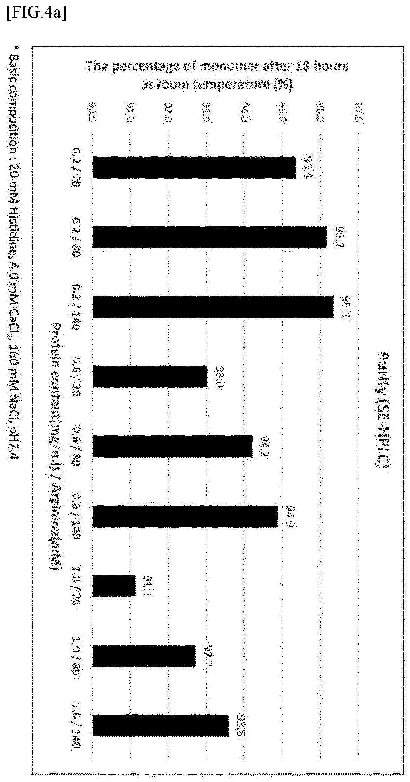

Each of the formulated samples was stored at room temperature for 18 hours and then subjected to SE-HPLC. As a result, as shown in FIG. 4a, it was confirmed that the purity increased as the concentration of the target protein decreased and as the arginine concentration increased.

### DOE design

Meanwhile, DOE (Design Of Experiments) was performed to more closely explore the optimal concentrations of arginine and the target protein. Two operating parameters, arginine concentration and ADAMTS protein concentration, were set as factors (X values) and purity (SE-HPLC) was set as the response (Y value), and then the DOE design was performed using the JMP^{®} 10.0 statistical program. Specifically, a response surface model (RSM) was used, and an axial point was set as a Central Composite Design (CCD) type. Finally, a total of 9 run conditions were designed, including "full factorial design 4 runs of 2 levels + center point 1 run + axis point 4 runs for 2 factors".

Arginine was added to the sample stock solution (1.1 mg/mL) according to DOE conditions, and the sample solution was diluted, left at room temperature (about 15 to 25°C) for 18 hours, and then subjected to SE-HPLC analysis.

DOE statistical analysis was performed through multiple regression analysis using stepwise regression, and the establishment of the model followed the response surface modeling method. The analysis was performed using the Fit model method of JMP, and the effects used in the model included two main effects, a 2-way interaction, and the square term of each main effect. As a stopping rule for removing insignificant factors and selecting significant terms, a P-value threshold was used (P value ≤ 0.25, direction: mixed).

As a result of the analysis, the R square of the model was 0.96 and the R square correction was 0.94. The P-value of ANOVA (analysis of variance) of the model was 0.0006, which is smaller than the significance level of 0.05, indicating the significance of this model.

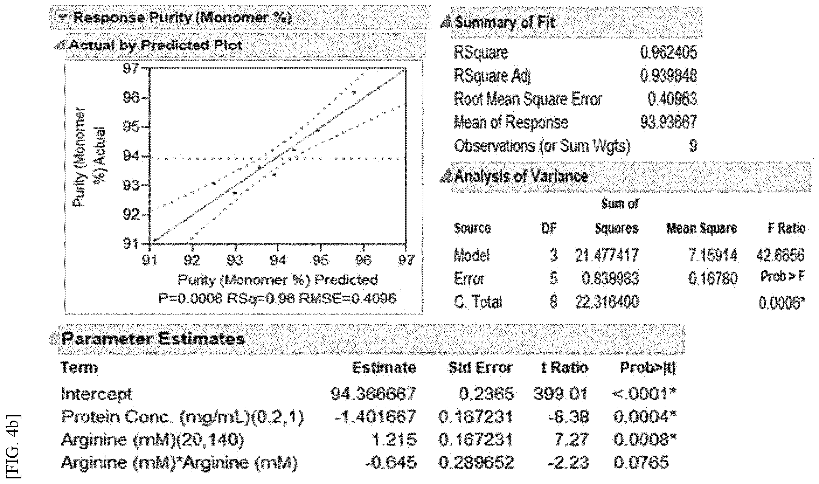

The factors that are considered significant by having a P-value lower than the significance level α=0.05 are the main effect, protein concentration, and the main effect, arginine concentration. The P-value for arginine*arginine curvature was 0.0765, which was close to the significance level of α=0.05 (FIG. 4b).

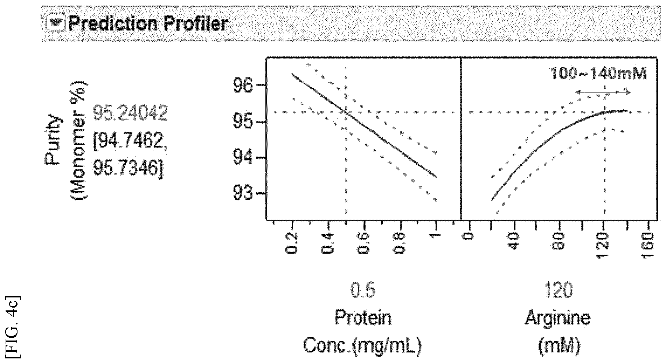

As a result of prediction profiler analysis, it could be seen that the monomer % value was the highest at around 120 mM arginine, indicating that 100 to 140 mM is the optimal concentration of arginine (FIG. 4c).

### Evaluation of quality of lyophilized composition depending on NaCl concentration

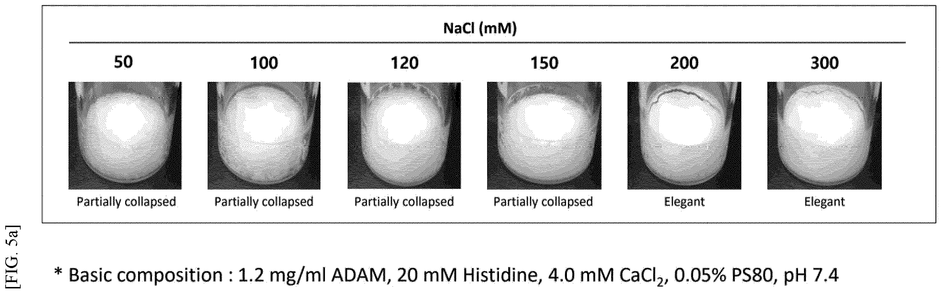

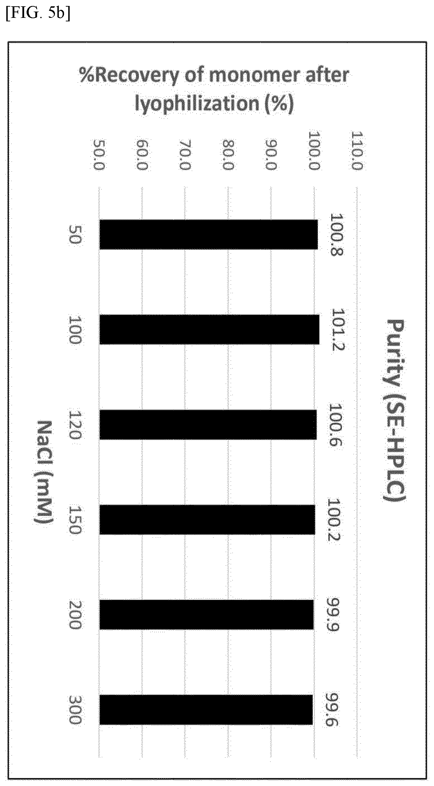

To a basic composition (pH 7.4) containing 1.2 mg/ml ADAMTS protein, 20 mM histidine, 4.0 mM CaCl₂ and 0.05% PS80, NaCl was added at concentrations of 50, 100, 120, 150, 200 and 300 mM. Each of the formulated samples was lyophilized, observed for the cake appearance, and subjected to SE-HPLC analysis. As a result, it was confirmed that, as the NaCl concentration increased, the cake appearance was more firm and better (FIG. 5a). However, as a result of SE-HPLC, no significant difference in purity depending on the NaCl concentration was observed (FIG. 5b).

### Evaluation of liquid phase stability depending on NaCl concentration

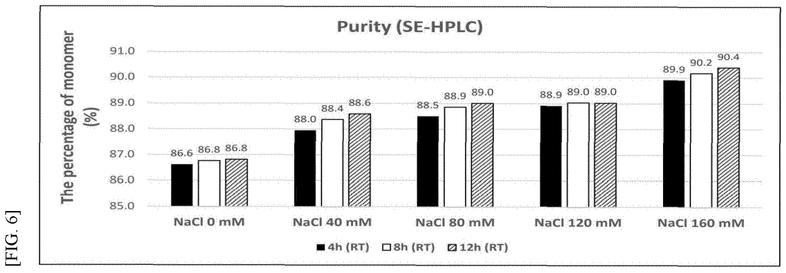

To a basic composition (pH 7.4) containing 1.2 mg/ml ADAMTS protein, 20 mM histidine, 4.0 mM CaCl₂ and 20 mM arginine, NaCl was added at concentrations of 0, 40, 80, 120 and 160 mM. Each of the formulated liquid samples was subjected to SE-HPLC analysis at 4, 8 and 12 hours during storage at room temperature. As a result, as shown in FIG. 6, it was confirmed that the higher the NaCl concentration, the better the liquid phase stability.

### Evaluation of quality of lyophilized composition depending on sugar concentration (120 mM NaCl)

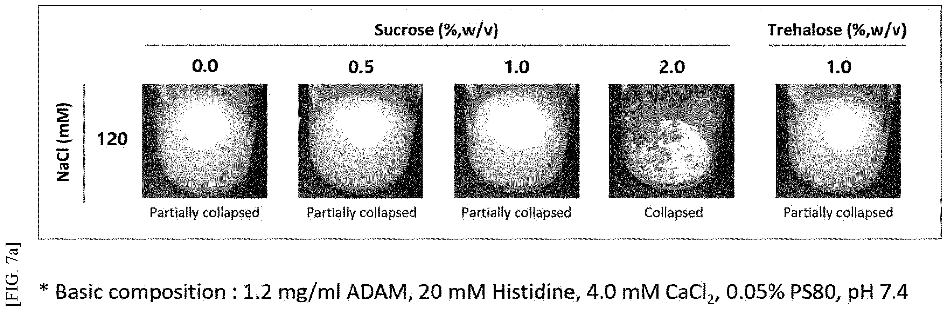

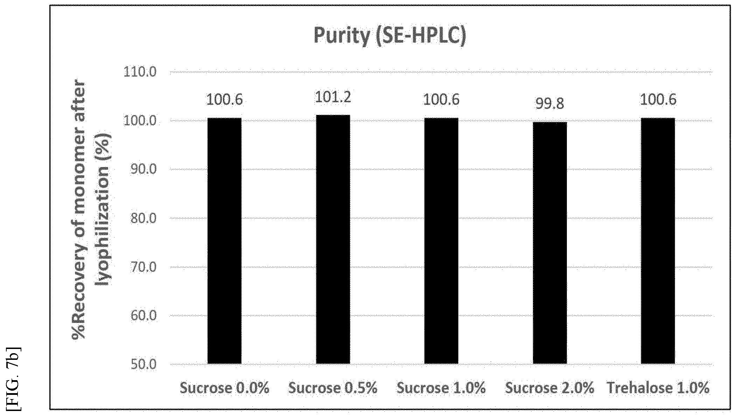

To a basic composition (pH 7.4) containing 1.2 mg/ml ADAMTS protein, 120 mM NaCl, 20 mM histidine, 4.0 mM CaCl₂ and 0.05% PS80, sucrose at 0.0, 0.5, 1.0 or 2.0% (w/v) sucrose or trehalose at 1.0% (w/v) was added. Each of the formulated samples was lyophilized, observed for the cake appearance, and subjected to SE-HPLC analysis. As a result, it was confirmed that, at 120 mM NaCl, no collapse occurred when 1.0% sucrose was added, but collapse occurred when 2.0% sucrose was added, indicating that the sucrose concentration at which collapse is initiated is between 1.0 and 2.0% (FIG. 7a). The formulation containing 1.0% trehalose showed an appearance similar to that of the formulation containing 1.0% sucrose (FIG. 7a). Meanwhile, as a result of SE-HPLC, it was confirmed that no significant difference in purity depending on the concentration of sugar added was observed, indicating that the purity was maintained even when collapse occurred.

### Evaluation of quality of lyophilized composition depending on content ratio between sucrose and bulking agent (120 mM NaCl)

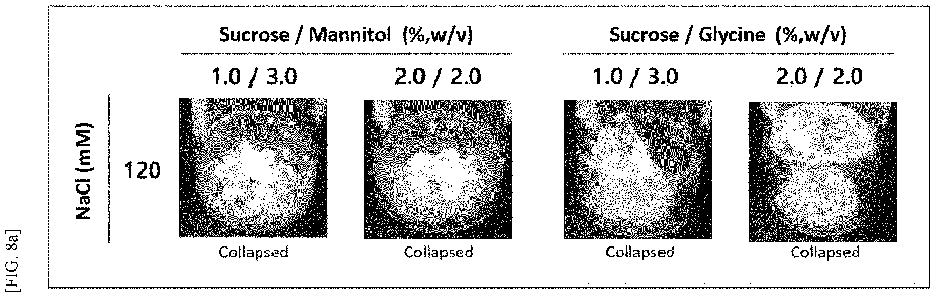

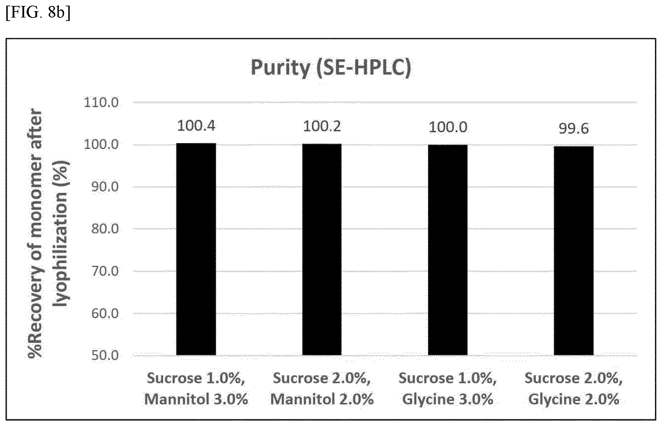

To a basic composition (pH 7.4) containing 1.2 mg/ml ADAMTS protein, 120 mM NaCl, 20 mM histidine, 4.0 mM CaCl₂ and 0.05% PS80, sucrose and a bulking agent (mannitol or glycine) were added at 1.0/3.0% (w/v) (1:3 content ratio) or 2.0/2.0% (w/v) (1:1 content ratio). Each of the formulated samples was lyophilized, observed for the cake appearance, and subjected to SE-HPLC analysis. As a result, it was confirmed that, when NaCl was fixed at 120 mM and sucrose and the bulking agent were added at a content ratio of 1:3 or 2:2, collapse occurred in all cases (FIG. 8a), and the results of SE-HPLC showed no significant difference between the samples (FIG. 8b).

### Evaluation of lyophilization quality depending on glycine concentration (120 mM NaCl)

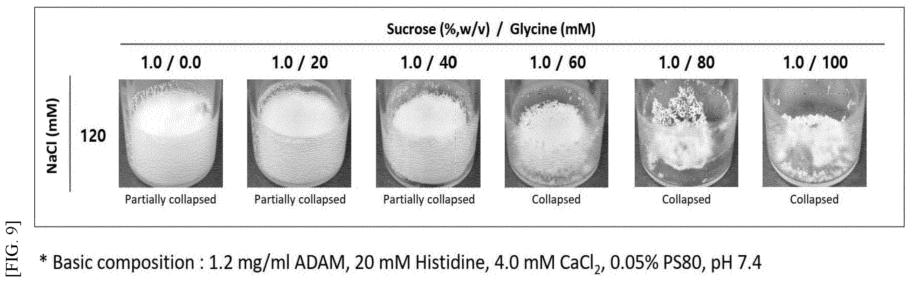

To a basic composition (pH 7.4) containing 1.2 mg/ml ADAMTS protein, 120 mM NaCl, 1.0% sucrose, 20 mM histidine, 4.0 mM CaCl₂ and 0.05% PS80, glycine was added at concentrations of 0, 20, 40, 60, 80 and 100 mM. Each of the formulated samples was lyophilized, observed for the cake appearance, and subjected to SE-HPLC analysis. As a result, it could be seen that, when 120 mM NaCl and 1.0% sucrose were fixedly added and the concentration of glycine was increased, the cake appearance was negatively affected, and when glycine was added at a concentration of 60 mM or more, the cake was completely collapsed (FIG. 9).

### Evaluation of lyophilization quality depending on arginine concentration (120 mM NaCl)

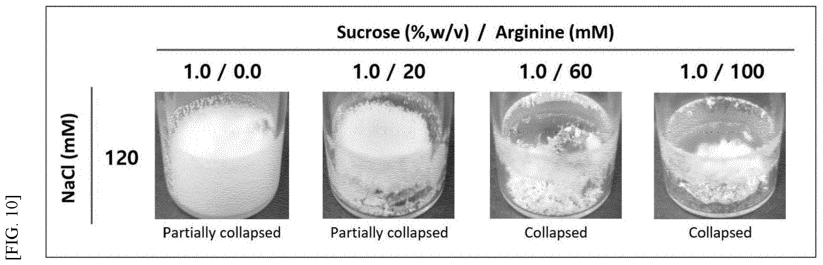

To a basic composition (pH 7.4) containing 1.2 mg/ml ADAMTS protein, 120mM NaCl, 1.0% sucrose, 20 mM histidine, 4.0 mM CaCl₂ and 0.05% PS80, arginine was added at concentrations of 0, 20, 60 and 100 mM. Each of the formulated samples was lyophilized, observed for the cake appearance, and subjected to SE-HPLC analysis. As a result, it was confirmed that, when 120 mM NaCl and 1.0% sucrose 1.0% were fixedly added and the arginine concentration was increased, the cake appearance was negatively affected, and when arginine was added at a concentration of 60 mM or more, the cake was completely collapsed (FIG. 10).

### Evaluation of lyophilization quality depending on content ratio between NaCl and sucrose (w/o arginine)

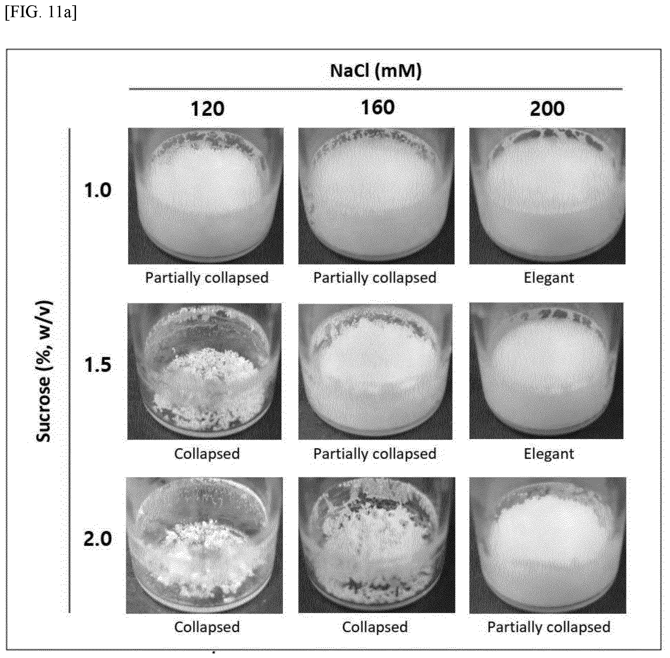

To a basic composition (pH 7.4) containing 1.2 mg/ml ADAMTS protein, 20 mM histidine, 4.0 mM CaCl₂ and 0.05% PS80, NaCl was added at concentrations of 120, 160 and 200 mM, and sucrose was added at concentrations of 1.0, 1.5 and 2.0% (w/v). Each of the formulated samples was lyophilized, observed for the cake appearance, and subjected to SE-HPLC analysis. As a result, it was shown that, as the NaCl concentration was increased and as the sucrose concentration was decreased, the cake appearance was better. Specifically, it was confirmed that, i) when 120 mM NaCl was added, collapse was initiated in a sucrose concentration range of 1.0 to 1.5%, ii) when 160 mM NaCl was added, collapse was initiated in a sucrose concentration range of 1.5 to 2.0%, and iii) when 200 mM NaCl was added, collapse was initiated at a sucrose concentration higher than 2.0% (FIG. 11a).

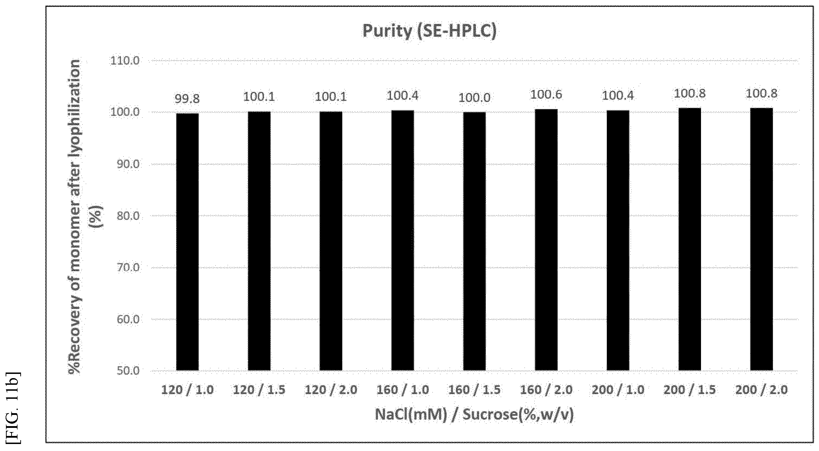

Meanwhile, as a result of SE-HPLC, it was confirmed that, when arginine was not added, no significant change depending on the content ratio between NaCl and sucrose was observed (FIG. 11b).

### Evaluation of lyophilization quality depending on content ratio between NaCl and sucrose (120 mM arginine)

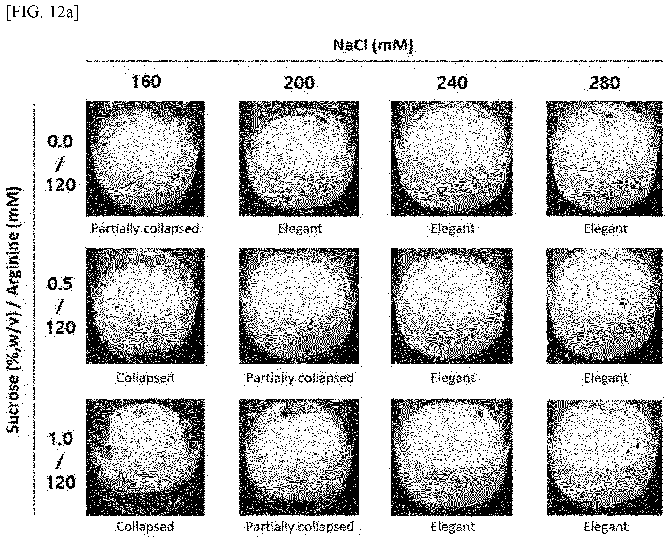

To a basic composition (pH 7.4) containing 0.5 mg/ml ADAMTS protein, 20 mM histidine, 4.0 mM CaCl₂, 0.05% PS80 and 120 mM arginine, NaCl was added at concentrations of 160, 200, 240 and 280 mM, and sucrose was added at concentrations of 0.0, 0.5 and 1.0% (w/v). Each of the formulated samples was lyophilized, observed for the cake appearance, and subjected to SE-HPLC analysis. As a result, it was confirmed that, when arginine was fixedly added at 120 mM, the cake appearance was firm as the NaCl concentration increased and as the sucrose concentration decreased. Specifically, it could be seen that, when i) 160 mM NaCl was added, collapse was initiated in a sucrose concentration range of 0.0 to 0.5% or more, ii) when 200 mM NaCl was added, collapse was initiated at a sucrose concentration higher than 1.0%, iii) when 240 mM NaCl was added, collapse was initiated at a sucrose concentration higher than 1.0%, and iv) when 280 mM NaCl was added, collapse was initiated at a sucrose concentration higher than 1.0% (FIG. 12a).

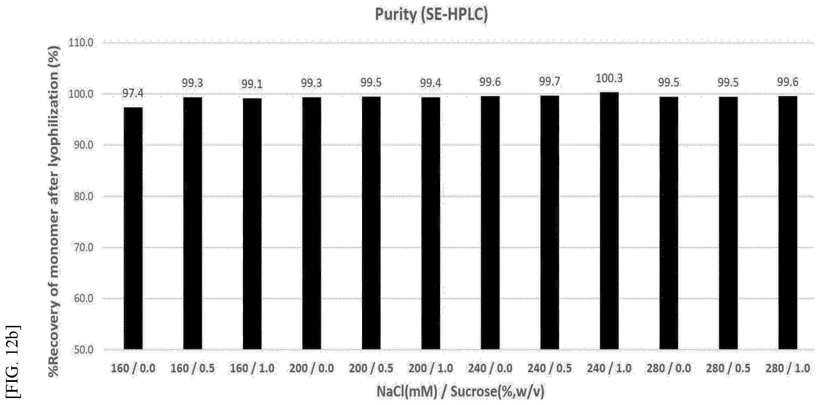

Meanwhile, as a result of SE-HPLC, it was confirmed that, when 120 mM arginine was added, no noticeable change in purity depending on the concentrations of NaCl and sucrose was observed, but the purity of the formulation containing 160 mM NaCl and no sucrose was 97.4%, which was relatively low compared to those of the other samples (FIG. 12b).

### Evaluation of lyophilization stability depending on mixing ratio of NaCl to sucrose (120 mM arginine)

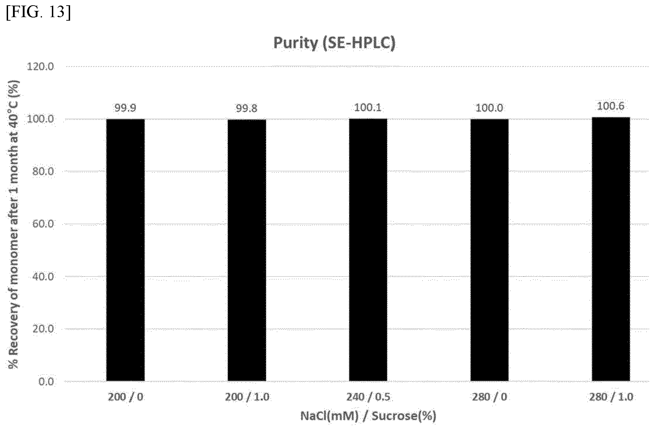

To a basic composition (pH 7.4) containing 0.5 mg/ml ADAMTS protein, 20 mM histidine, 4.0 mM CaCl₂, 0.05% PS80 and 120 mM arginine, NaCl was added at concentrations of 200, 240 and 280 mM, and sucrose was added at concentrations of 0.0, 0.5 and 1.0% (w/v). Each of the formulated samples was lyophilized and the accelerated stability (40°C) thereof was evaluated. As a result, it was confirmed that, in the presence of 120 mM arginine, the purity after 1 month at 40°C depending on the mixing ratio of NaCl to sucrose did not significantly change compared to that in the initial stage (FIG. 13).

### Prevention of agitation-induced aggregation depending on polysorbate 80 concentration

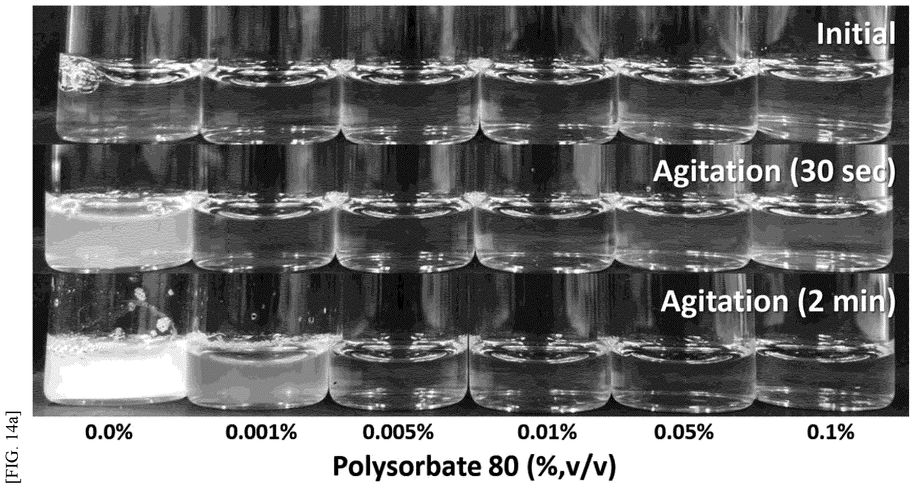

To a basic composition (pH 7.4) containing 1.2 mg/ml ADAMTS protein, 20 mM histidine, 4.0 mM CaCl₂, 120mM NaCl and 1.0% sucrose, polysorbate 80 was added at concentrations of 0.0, 0.001, 0.005, 0.01, 0.05 and 0.1% (v/v). Each of the formulated liquid samples was vortexed to generate artificial shear stress, and then analyzed (appearance, turbidity, and SE-HPLC). Samples taken 30 seconds and 2 minutes after the start of agitation were analyzed. As a result of analyzing the appearance after 2 minutes of agitation, it was confirmed that, when polysorbate 80 was added at a concentration of 0.005% or more, the sample became clear and transparent (FIG. 14a).

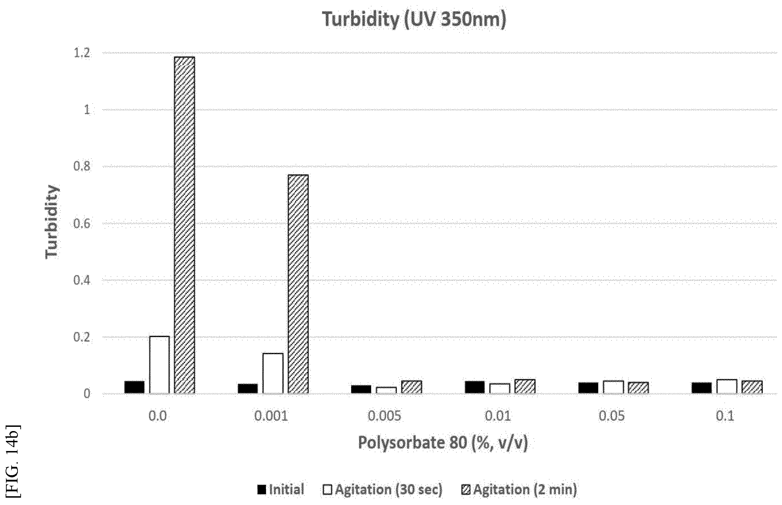

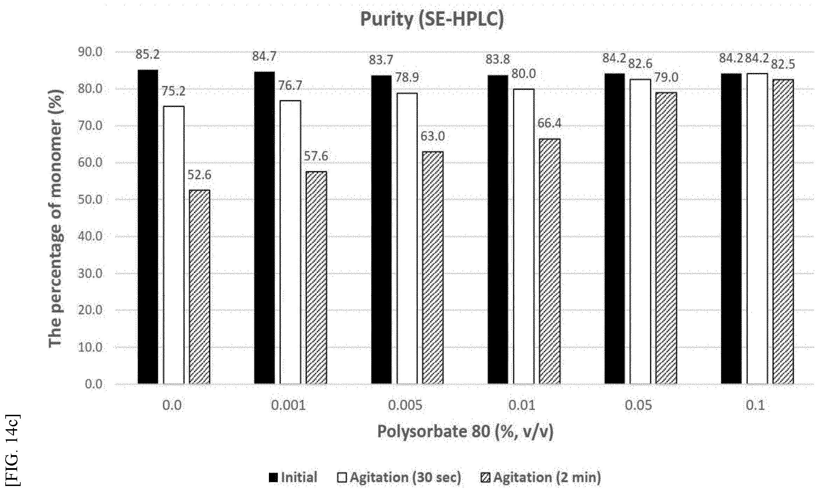

Meanwhile, it could be seen that, when polysorbate 80 was added at a concentration of 0.005% or more, there was no change in turbidity compared to that in the initial stage, indicating that agitation-induced aggregation did not occur (FIG. 14b). In addition, as a result of SE-HPLC, it was confirmed that, when polysorbate 80 was added at 0.05% or more, the purity decreased by 5% or less compared to that in the initial stage (FIG. 14c).

### Evaluation of liquid phase stability depending on polysorbate 80 concentration

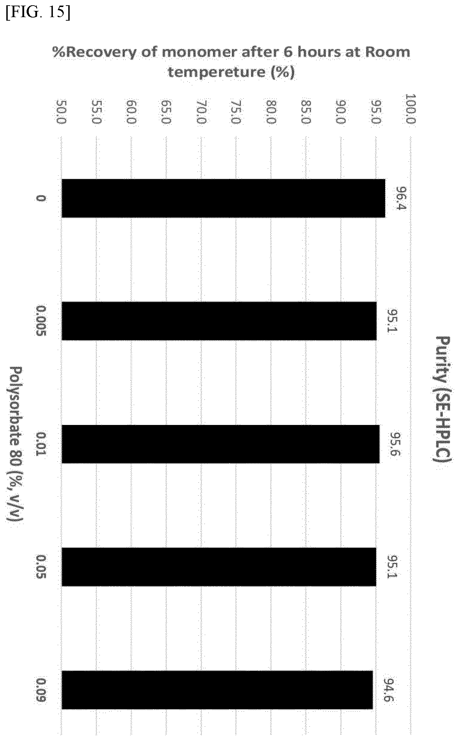

To a basic composition (pH 7.4) containing 0.5 mg/ml ADAMTS protein, 20 mM histidine, 4.0 mM CaCl₂, 120 mM NaCl and 1.0% sucrose, polysorbate 80 was added at concentrations of 0.0, 0.005, 0.01, 0.05 and 0.09% (v/v). Each of the formulated liquid samples was stored at room temperature for 6 hours and then analyzed by SE-HPLC. As a result, it was confirmed that, as the polysorbate 80 concentration increased, the liquid phase stability (purity) decreased slightly (FIG. 15).

### Evaluation of lyophilization quality depending on polysorbate 80 concentration

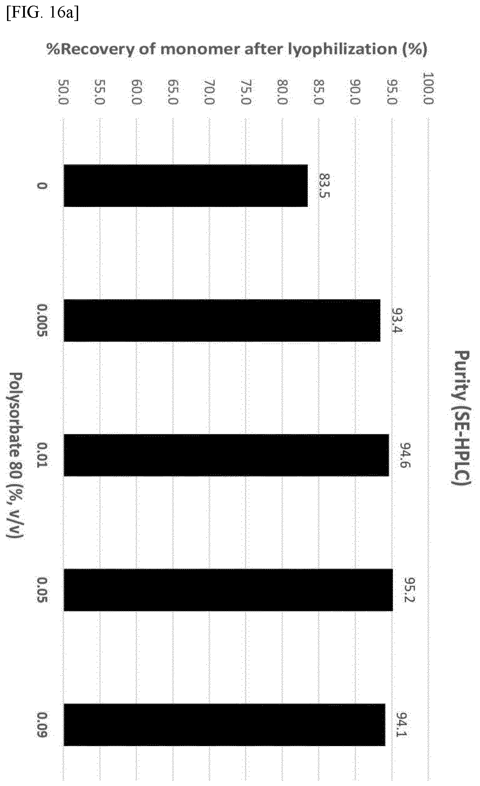

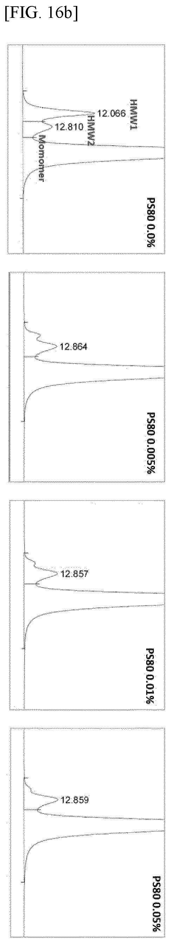

To a basic composition (pH 7.4) containing 0.5 mg/ml ADAMTS protein, 20 mM histidine, 4.0 mM CaCl₂, 120mM NaCl and 1.0% sucrose, polysorbate 80 was added at concentrations of 0.0, 0.005, 0.01, 0.05 and 0.09% (v/v). Each of the formulated liquid samples was lyophilized and then analyzed by SE-HPLC. As a result, it was confirmed that, as the polysorbate 80 concentration increased, the effect of preventing purity from being lost due to the lyophilization process increased, and when polysorbate 80 was added at a concentration of 0.01% or more, a monomer recovery of 94% or more was found (FIG. 16a). In addition, as the polysorbate 80 concentration increased, the formation of higher-order aggregates was effectively blocked (FIG. 16b).

### Evaluation of thermal unfolding and thermal aggregation depending on pH

Diafiltration buffers containing a basic composition (containing 20 mM histidine, 4 mM CaCl₂ and 0.05% PS80) and having different pHs of 6.0, 6.5, 7.0, 7.2 and 7.4 were prepared, and then subjected to buffer exchange with a solution containing ADAMTS protein, thus preparing samples. Amicon^{®} Ultra-15 Centrifugal Filter Units 30K was used, and centrifugation was performed at 3,000 rpm under refrigeration conditions.

The buffer-exchanged samples had a common composition containing 10 mg/ml ADAMTS protein, 20 mM histidine, 4 mM CaCl₂ and 0.05% PS80, and the pHs thereof were 6.0, 6.5, 7.0, 7.2 and 7.4, respectively.

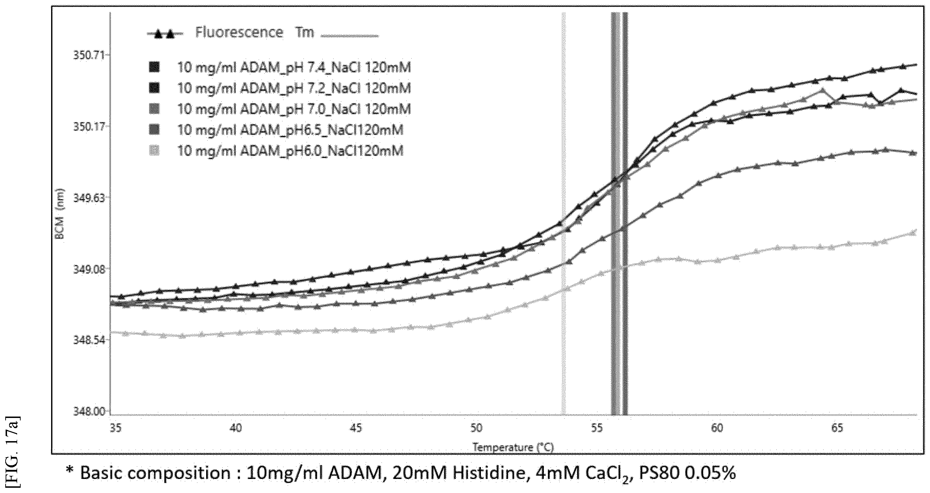

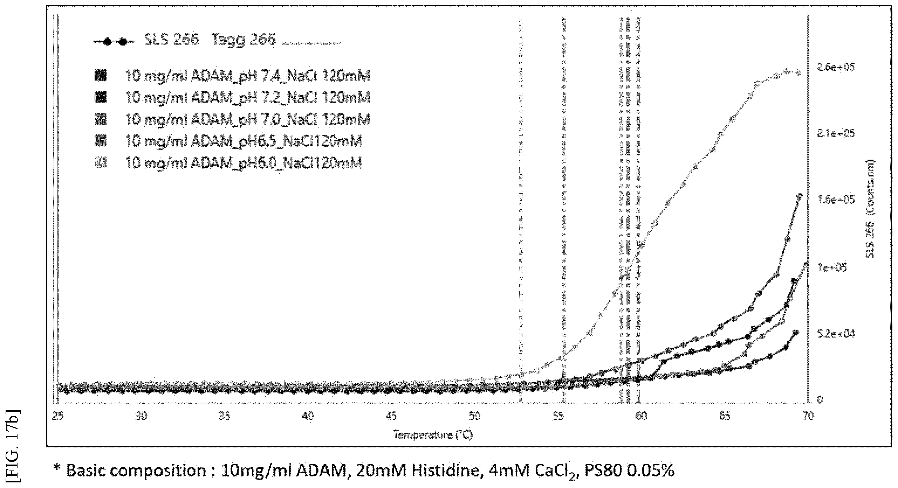

As a result of subjecting the formulated liquid samples to Tₘ and T_{agg} analysis using the UNCLE system, it was confirmed that the thermal unfolding (Tₘ) value, which is proportional to the structural thermal stability of the protein, increased as the pH increased, and the sample at pH 6.0 showed a lower Tm value than the other samples (FIG. 17a). It was confirmed that the thermal aggregation (T_{agg}) value, which reflects the degree of unstable aggregation, increased as the pH increased, and the samples at pH 6.0 and pH 6.5 showed lower T_{agg} values than the other samples (FIG. 17b).

### Evaluation of lyophilization quality depending on CaCl₂ concentration

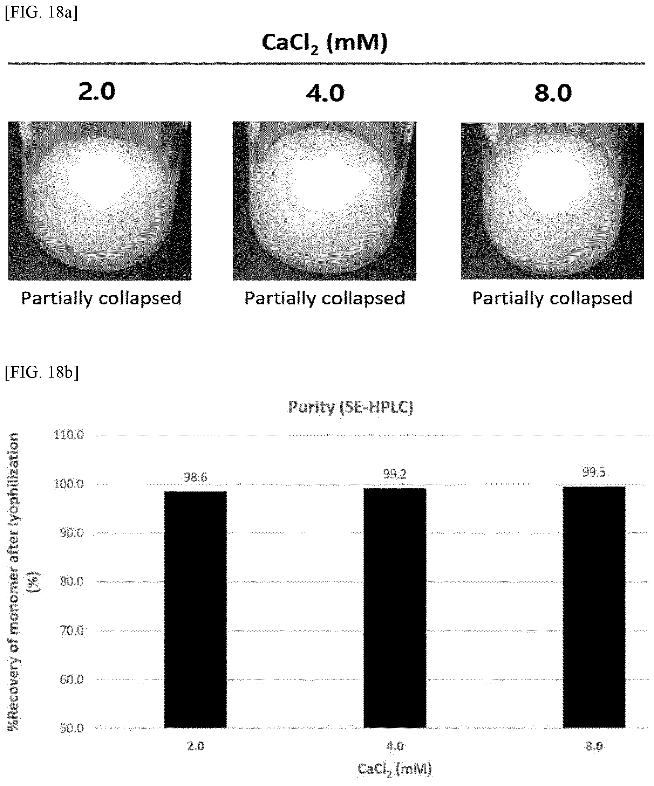

To a basic composition (pH 7.4) containing 1.2 mg/ml ADAMTS protein, 20 mM histidine, 120 mM NaCl, 1.0% sucrose and 0.05% PS80, CaCl₂ was added at concentrations of 2.0, 4.0 and 8.0 mM. Each of the formulated samples was lyophilized and subjected to SE-HPLC analysis. As a result, it was confirmed that the samples showed a partially collapsed appearance regardless of the CaCl₂ concentration, (FIG. 18a). As a result of SE-HPLC analysis, it was confirmed that the purity increased as the CaCl₂ concentration increased (FIG. 18b).

### Example 2: Further optimization of components for formulation of ADAMTS13 protein

### Evaluation of lyophilization quality depending on mixing ratio of NaCl to sucrose

To a basic composition (pH 7.4) containing 0.36 mg/ml ADAMTS protein, 20 mM histidine, 4 mM CaCl₂, 120 mM L-Arg and 0.05% PS80, NaCl was added at concentrations of 100, 150, 200, 250, 300, 350 and 400 mM, and sucrose was added at concentrations of 0, 0.5, 1 and 1.5% (w/v). Each of the formulated samples was lyophilized, observed for the cake appearance, and analyzed by SE-HPLC for the purities before and after the lyophilization process. As a result, as shown in Table 2 below, it was confirmed that no significant change in the purity depending on the content ratio between NaCl and sucrose was observed, indicating that high purity was maintained in all tested ranges.

**[Table 2] Purity recovery (%) after lyophilization**

| | | **NaCl (mM)** | | | | | | |
|---|---|---|---|---|---|---|---|---|
| | | **100** | **150** | **200** | **250** | **300** | **350** | **400** |
| **Sucrose (%)** | **0** | 100.6 | 100.8 | 100.3 | 100.1 | 99.4 | 99.8 | 100.0 |
| | **0.5** | 100.7 | 100.9 | 100.3 | 100.2 | 99.9 | 99.9 | 99.9 |
| | **1** | 100.7 | 100.9 | 100.4 | 100.2 | 100.2 | 99.9 | 99.9 |
| | **1.5** | 100.1 | 101.0 | 100.1 | 100.2 | 100.2 | 100.0 | 100.0 |

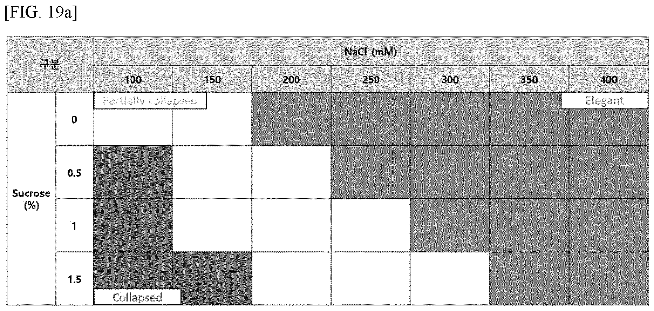

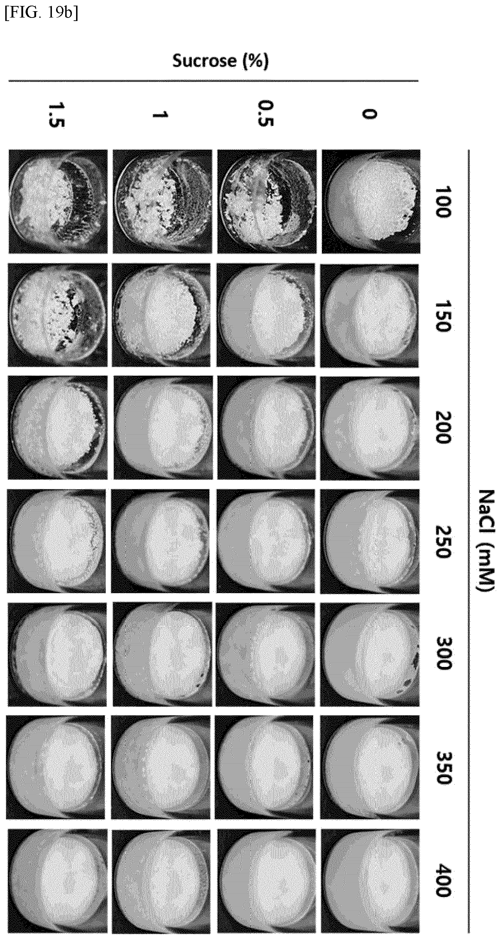

However, as shown in FIGS. 19a and 19b, it was confirmed that the cake appearance was better as the NaCl concentration increased and as the sucrose concentration decreased.

### Evaluation of lyophilization quality depending on high-concentration sucrose treatment

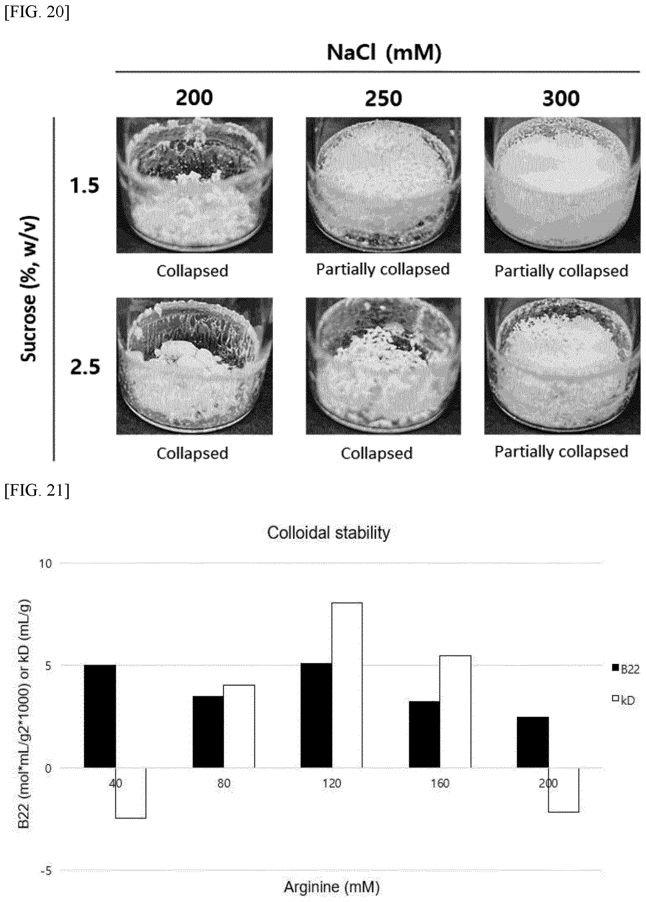

To a basic composition (pH 7.4) containing 0.36 mg/ml ADAMTS protein, 20 mM histidine, 4 mM CaCl₂, 120 mM L-Arg and 0.05% PS80, NaCl was added at concentrations of 200, 250 and 300 mM, and sucrose was added at concentrations of 1.5 and 2.5 %. Each of the formulated samples was lyophilized and observed for the cake appearance. As a result, as shown in FIG. 20, it was confirmed that, as the NaCl concentration increased and as the sucrose concentration decreased, the cake appearance was better.

### Evaluation of colloidal stability depending on arginine concentration (B₂₂, kD)

To a basic composition (pH 7.4) containing 0.36 mg/ml ADAMTS protein, 20 mM histidine, 4 mM CaCl₂, 280 mM NaCl, 1% sucrose and 0.05% PS80, arginine was added at concentrations of 40, 80, 120, 160 and 200 mM. Each of the formulated liquid samples was analyzed for colloidal stability (B₂₂, kD) using the UNCLE system. Higher positive B₂₂ and kD values mean that the protein is well dispersed without aggregation, and as shown in FIG. 21, it was confirmed that the closer to 120 mM arginine, the better the colloidal stability.

### Evaluation of lyophilization quality depending on arginine concentration

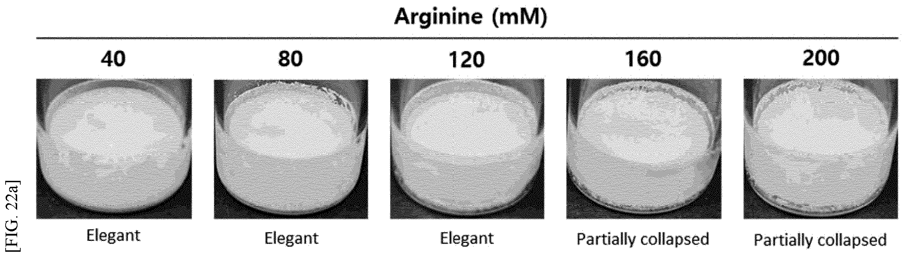

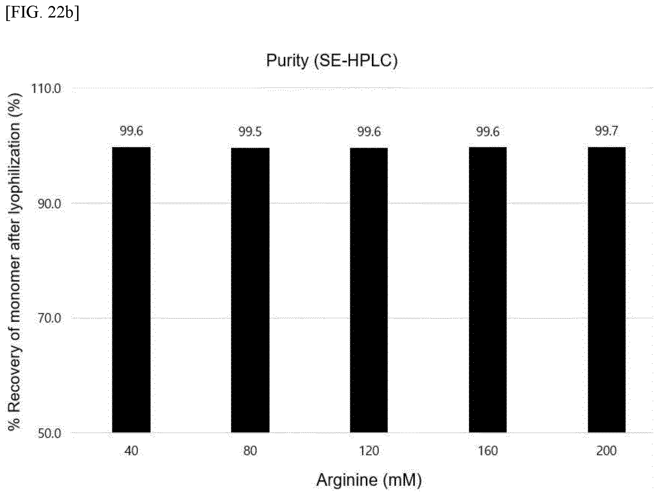

To a basic composition (pH 7.4) containing 0.36 mg/ml ADAMTS protein, 20 mM histidine, 4 mM CaCl₂, 280 mM NaCl, 1% sucrose and 0.05% PS80, arginine was added at concentrations of 40, 80, 120, 160 and 200 mM. Each of the formulated samples was lyophilized, observed for the cake appearance, and analyzed for the purity by SE-HPLC. As a result, it was confirmed that the cake appearance was good when the arginine concentration was 80 mM and 120 mM (FIG. 22a), but no significant difference in the purity depending on the arginine concentration was observed (FIG. 22b).

### Storage time and temperature stability for GC1126A final liquid formulation

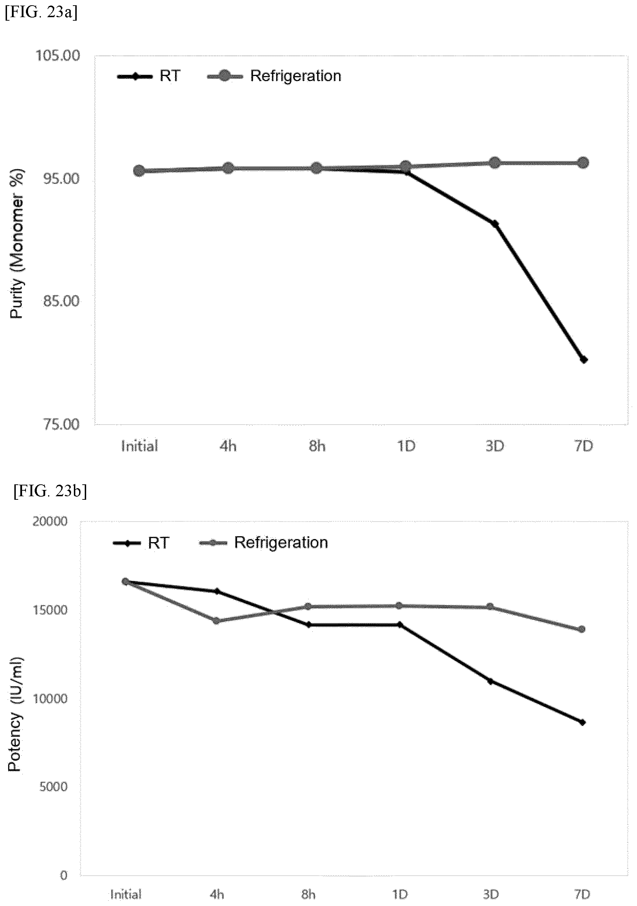

The formulated liquid sample (pH 7.4) containing 0.36 mg/ml ADAMTS protein, 20 mM histidine, 4 mM CaCl₂, 120 mM L-Arg, 280 mM NaCl, 1% sucrose and 0.05% PS80 was stored under room temperature (25°C) or refrigeration (4°C) conditions, and then time-dependent changes of the samples were examined by performing analysis of purity (SE-HPLC), potency (ADAMTS13 activity), protein concentration (UV) and turbidity. As a result of analyzing the purity, it was confirmed that, on and after 3 days of storage at room temperature, there was a decrease in purity of about 4% compared to that in the initial stage, and on 7 days of storage, a decrease in purity of about 15% compared to that in the initial stage, and until 7 days of refrigeration storage, no time-dependent change was observed (FIG. 23a).

As a result of observing the change in the potency, it was confirmed that, on 7 days of storage at room temperature, there was a decrease in potency of about 50% compared to that in the initial stage, and considering the deviation of the potency analysis, no significant time-dependent change was observed with time until 7 days of refrigeration storage (FIG. 23b).

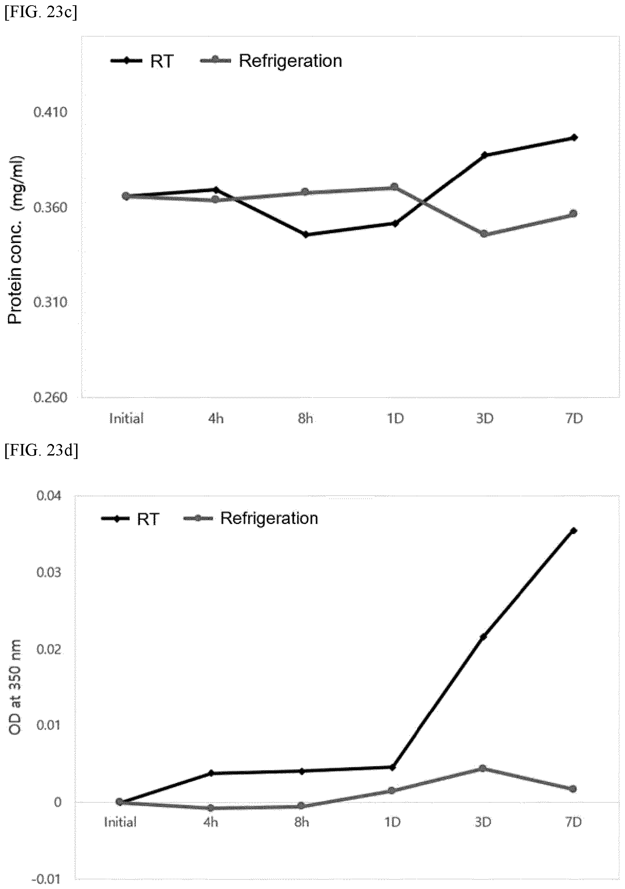

The protein concentration increased from day 3 of storage at room temperature compared to that in the initial stage, which is believed to be the result of an increase in UV absorbance due to an increase in opacity of the sample due to aggregation of particles. No time-dependent change was observed up to 7 days of refrigeration storage (FIG. 23c).

Meanwhile, the turbidity of the final liquid formulation tended to increase from 3 days of room-temperature storage, but no time-dependent change was observed until 7 days of refrigeration storage (FIG. 23d).

### Optimization of NaCl and sucrose concentrations

To a basic composition (pH 7.4) containing 0.5 mg/ml ADAMTS protein, 20 mM histidine, 4 mM CaCl₂, 120 mM L-Arg and 0.05% PS80, NaCl was added at concentrations of 200, 240 and 280 mM, and sucrose was added at concentrations of 0, 0.5 and 1%.

Design Of Experiments (DOE) was performed to explore the optimal concentrations of NaCl and sucrose. After setting two operating parameters, NaCl and sucrose concentration, as factors (X values) and setting purity (SE-HPLC) and potency (ADAMTS13 activity) as responses (Y values), DOE design was performed using the JMP^{®} 10.0 statistical program. For the DOE design, a full factorial design was used, and two central points were included. Finally, a total of 6 conditions were designed, including "full factorial design 4 runs of 2 levels + center point 2 runs".

**[Table 3]**

| **Run** | **X** | |
|---|---|---|
| | **NaCl (mM)** | **Sucrose** |
| 1 | 280 | 0 |
| 2 (central point) | 240 | 0.5 |
| 3 | 280 | 1 |
| 4 | 200 | 0 |
| 5 | 200 | 1 |
| 6 (central point) | 240 | 0.5 |

A formulation buffer solution was prepared according to the pre-designed conditions and mixed with the stock solution, thus preparing a final stock solution. 1 ml of the prepared formulation was dispensed into each 3-ml vial and lyophilized, and the lyophilized sample was refrigerated (5°C) and analyzed for quality after 6 months.

DOE statistical analysis was performed through multiple regression analysis using stepwise regression. For analysis, the "Fit model" method of JMP was used, and the effects used in the model included two main effects, a 2-way interaction, and the square term of each main effect. As a stopping rule for removing insignificant factors and selecting significant terms, a P-value threshold was used (P value ≤ 0.25, direction: mixed).

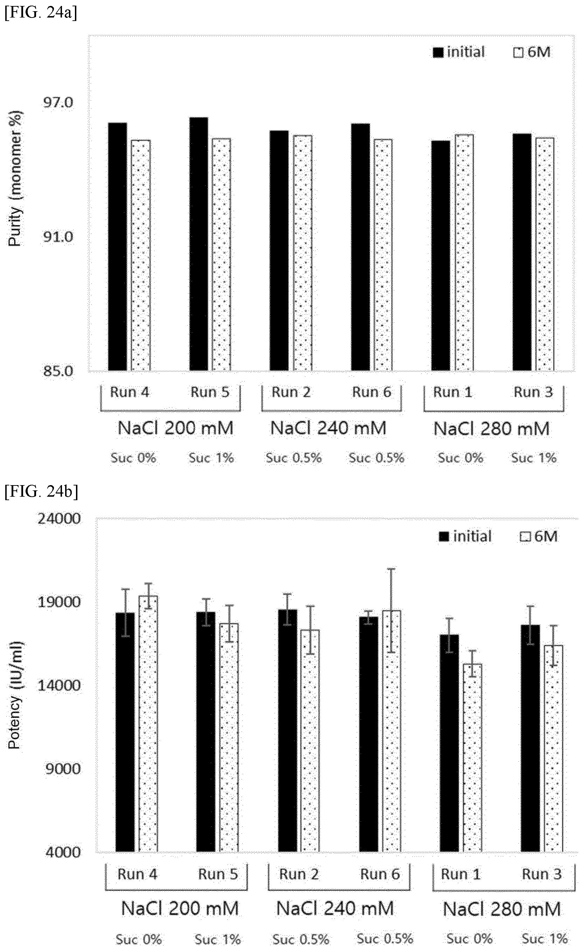

As a result of the analysis, good purity was shown for 6 months under all experimental conditions, and no difference in purity depending on the concentrations of NaCl and sucrose was observed (FIG. 24a). In addition, the potency after 6 months compared to the initial potency of each sample was within the analysis deviation and did not show a significant difference (FIG. 24b).

### Example 3: Construction of ADAMTS13 protein variants

The present inventors used a random mutagenesis method to construct human ADAMTS13 variants capable of efficiently evading ADAMTS13 neutralizing antibodies possessed by aTTP patients. In order to examine whether a variant having mutation in the MDTCS portion or the domain S can evade ADAMTS13 neutralizing antibodies, Fabs for antibodies recognizing different epitopes for human ADAMTS13 were constructed using the HuCAL system (Bio-Rad), and 16 Fabs having excellent binding affinity to human ADAMTS13 were selected. Regarding the binding region of each antibody, as shown in FIG. 25c, Ab 4-16 binds specifically to domain S, Ab 67 binds specifically to the MDTCS portion, and Ab 66 binds specifically to the C-terminal portion (domain TSP-2 to domain TSP-8).

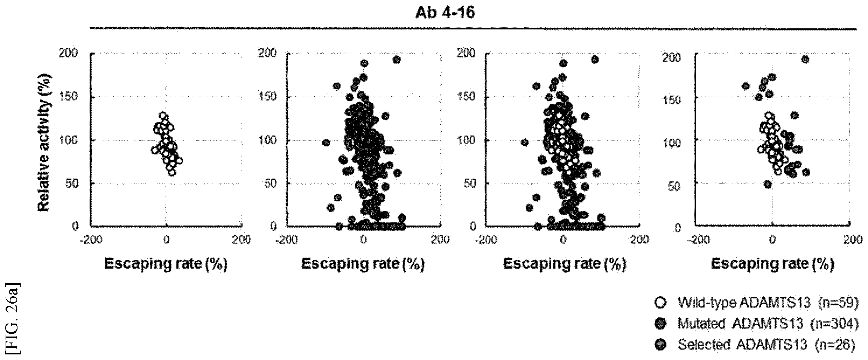

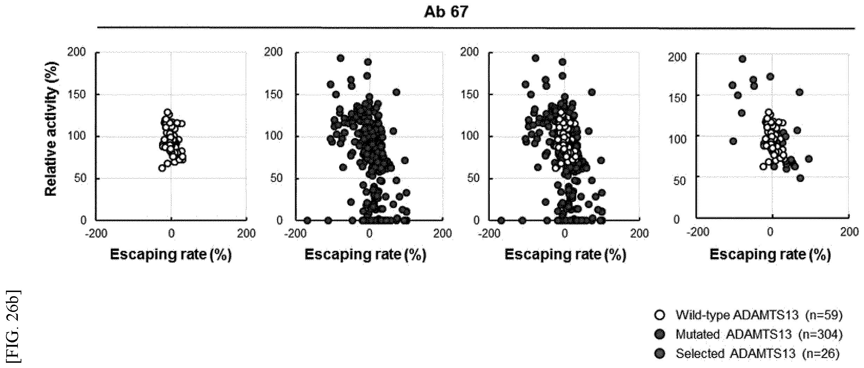

Evaluation of the variants was performed through analysis of the relative results for the wild-type ADAMTS 13 construct and the non-mutated or silent mutated variants (hereinafter referred to as wild-type clones) having the same amino acid sequence as the wild-type ADAMTS13 among variants produced through random mutagenesis. For a total of 59 wild-type clones, the activity and the binding affinities of Ab 4-16 and Ab 67 were analyzed. The analysis results for each wild-type clone were relativized based on the resultant values for the wild-type ADAMTS13 construct, and the results are shown in FIG. 26. FIG. 26 shows the escaping rates and relative activities of wild-type ADAMTS13 (n=59) containing conservative amino acid substitutions, mutated ADAMTS13 (n=304), and selected ADAMTS13 (n=26) for antibodies Ab 4-16 and Ab 67. The wild-type clones showed a difference from the results values for the wild-type ADAMTS13 construct despite having no mutation. Among the mutated ADAMTS13 (n=304), 26 mutated ADAMTS13 having values equal to or higher than the specific escaping rate or relative activity shown in Table 4 below were selected.

Specifically, the Ab 4-16 escaping rate of the mutated ADAMTS13 ranged from -29.5% to 33.4%, and the Ab 67 escaping rate thereof ranged from -24.4% to 30.5%, and the relative activity thereof ranged from 62.7% to 128.9% (Table 4). As a result of applying a normal distribution using the three-sigma rule, the Ab 4-16 escaping rate ranged from -34.1% to 38.5%, the Ab 67 escaping rate ranged from - 38.5% to 42.3%, the relative activity ranged from 47.0% to 145.2%, and almost all results for the WT clones were expected to be within this distribution. Therefore, in the selection of variants, based on the maximum value of three-sigma, an Ab 4-16 escaping rate of more than 38.5%, an Ab 67 escaping rate of more than 42.3%, or a relative activity of 47.0% or more was applied as selection criteria.

**[Table 4] Escaping rate for neutralizing antibody Ab4-16 and Ab-67, and relative activity**

| Category | Ab 4-16 escaping rate | Ab 67 escaping rate | Relative activity |
|---|---|---|---|
| Minimum value | -29.5% | -24.4% | 62.7% |
| Maximum value | 33.4% | 30.5% | 128.9% |
| Mean | 2.2% | 1.9% | 96.1% |
| Standard deviation | 12.1% | 13.5% | 16.4% |
| mean - 3 standard deviations | -34.1% | -38.5% | 47.0% |
| mean + 3 standard deviations | 38.5% | 42.3% | 145.2% |

### Selection of variants that evade ADAMTS13 neutralizing antibodies and have higher activity than that of wild-type ADAMTS13

After transfection of each of WT clones and variants having amino acid mutations into cells, the amount of protein present in each culture medium was measured, and neutralizing antibody binding affinity and activity assays were performed for 304 variants having a protein expression concentration of 50 ng/mL or more. It was confirmed that variants with amino acid mutations had a diverse distribution in neutralizing antibody binding affinity or relative activity compared to the WT clones. Among these variants, 26 variants satisfying the selection criteria were finally selected. It was confirmed that 18 of the 26 variants had mutations in the domain S, and in particular, 13 variants (1C03, 1G07, 2B01, 2B02, 3B05, 3G04, 5C09, 5G08, 6B12, 7A02, 8C04, 8D01, and 8F01) having mutations in the domain S had high Ab 4-16 escaping rates, and five variants (7E01, 7G08, 8C02, 8D01, and 8D05) showed high relative activity (Table 5). Six variants had mutations in the domain D, and thereamong, five variants (46, 3A06, 4C07, 4E11, and 4H07) showed high Ab 67 escaping rates. In addition, 6 variants having mutations in the domain M, 2 variants having mutations in the domain C, and 2 variants having mutations in the domain T were identified. Repeated reproducibility of the results was tested through three repeated experiments for the 26 selected variants, and 12 variants that continuously maintained superiority over the WT clones in the repeated tests were selected as subjects for the *in vitro* efficacy test. Among the 12 variants, 1C03, 2B01, 2B02, 3B05, 5C09, 5G08, 7A02 and 8D01 were selected because of their excellent Ab4-16 antibody escaping rates, and 4C07, 4E11 and 4H07 were selected because of their excellent Ab67 escaping rates. Finally, 8D05 was selected based on its superior relative activity.

In order to examine the ability of the selected 12 variants to evade additional neutralizing antibodies, the present inventors examined whether these variants could evade neutralizing antibodies Ab4-20, Ab60, Ab61, Ab64 and Ab65 in addition to the Ab4-16 and Ab67 antibodies used for screening. Among the 12 variants, 8 variants (1C03, 2B01, 2B02, 3B05, 5C09, 5G08, 7A02, and 8D01) excluding 8D05 among 9 variants having amino acid mutations in the domain S exhibited high escaping rates from Ab4-16 and Ab4-20 constructed based on the ADAMTS13 autoantibody sequence possessed by aTTP patients, and also showed an excellent ability to evade Ab60 and Ab61. On the other hand, variants 4C07, 4E11, and 4H07 having amino acid mutations in the domain D had high escaping rates from Ab67 (FIG. 27 and Table 6).

**[Table 5]**

| Selection of 26 variants having high escaping rates from Ab4-16 and Ab67 neutralizing antibodies or excellent relative activity | | | | | |
|---|---|---|---|---|---|
| Variant ID | Domain with mutation | Mutated amino acid residue | Ab4-16 antibody escaping rate | Ab67 antibody escaping rate | Relative activity |
| 46 | M,D | L85F, P317H | 46.30% | 53.90% | 66.80% |
| 1C03 | S | S612Y | 48.00% | 10.40% | 104.60% |
| 1G07 | M,C,S | V282A, A465D, D672V | 41.40% | 57.90% | 63.80% |
| 2B01 | S | D635V | 87.40% | 5.40% | 62.20% |
| 2B02 | C,S | R452I, S612Y | 69.80% | 15.30% | 88.80% |
| 3A06 | M,D,T | R278I, A334T, D427N | 42.60% | 58.50% | 62.90% |
| 3B05 | S | P618S | 66.00% | 48.40% | 71.20% |
| 3E01 | M | T135I | 52.30% | 36.00% | 60.00% |
| 3G04 | M,S | V126M, A567S, E651D | 42.00% | 29.40% | 103.50% |
| 3H12 | T | N413D | -69.00% | -104.60% | 162.40% |
| 4C07 | D | A334V | -6.60% | 72.30% | 153.10% |
| 4E11 | D | A314T | 10.30% | 96.40% | 71.90% |
| 4H03 | M, D | F93V, K364R, E376D | 40.30% | 37.60% | 65.70% |
| 4H07 | D | N308K | 30.20% | 64.60% | 106.40% |
| 5C09 | S | S612F | 60.90% | -10.50% | 88.50% |
| 5G08 | S | Q656H | 41.40 | 26.00% | 91.90% |
| 6B12 | S | G607R | 43.90% | -101.60% | 93.80% |
| 7AO2 | S | S612F, G624D | 57.00% | -80.50% | 128.50% |
| 7E01 | S | R589Q | -36.60% | -89.70% | 149.90% |
| 7G08 | S | Q650H, Q656R | -25.20% | -47.60% | 160.70% |
| 8C02 | S | 1643F | -19.20% | -50.90% | 168.10% |
| 8C04 | S | I585N, Y658H | 51.30% | 34.80% | 69.00% |
| 8C12 | S | V630L, D654G, E664N | -12.00% | 73.20% | 48.10% |
| 8D01 | S | R589Q, K608M, M609L, G624C, I655V | 85.10% | -78.30% | 193.60% |
| 8D05 | S | P578L | -1.20% | -5.70% | 172.30% |
| 8F01 | S | I585M | 45.20% | 34.40% | 99.40% |

**[Table 6]**

| Evaluation of single neutralizing antibody escaping rates and relative activities of 12 selected variants | | | | | | | | | |
|---|---|---|---|---|---|---|---|---|---|
| **Variant ID** | **Mutation position** | **Neutralizing antibody escaping rate (%)** | | | | | | | **Relative activity** |
| | | **AAb4-16** | **AAb4-20** | **AAb60** | **AAb61** | **AAb64** | **AAb65** | **AAb67** | |
| 1C03 | S612Y | 57.5% | 59.4% | 97.2% | 94.7% | 5.2% | -11.0% | 15.1% | 103.7% |
| 2B01 | D635V | 82.9% | 88.6% | 92.4% | 97.4% | 96.3% | 8.1% | 7.9% | 66.8% |
| 2B02 | R452I, S612Y | 65.4% | 61.1% | 97.3% | 95.5% | 0.9% | -15.0% | 15.1% | 112.6% |
| 3B05 | P618S | 51.3% | -6.5% | 24.9% | 39.4% | 19.4% | -63.8% | 37.4% | 77.3% |
| 4C07 | A334V | 4.2% | -5.0% | -3.3% | -6.9% | -2.2% | -4.7% | 78.5% | 152.2% |
| 4E11 | A314T | 16.6% | -1.3% | 2.7% | 3.2% | 5.4% | -0.8% | 98.9% | 78.3% |
| 4H07 | N308K | 27.9% | 1.5% | 11.0% | 16.4% | 17.0% | 12.8% | 61.3% | 94.7% |
| 5C09 | S612F | 64.6% | 54.7% | 97.3% | 95.2% | 7.8% | -1.6% | 11.8% | 104.8% |
| 5G08 | Q656H | 47.0% | 7.2% | 32.1% | 46.2% | 27.4% | -51.4% | 30.3% | 95.9% |
| 7A02 | S612F, G624D | 57.1% | 55.4% | 97.6% | 95.5% | 1.9% | -26.2% | -12.9% | 125.3% |
| 8D01 | R589Q, K608M, M609L, G624C, 1655V | 90.1% | 98.5% | 95.2% | 94.1% | 20.0% | -95.9% | 7.3% | 127.0% |
| 8D05 | P578L | -9.3% | -43.1% | 3.2% | 15.9% | -0.9% | -116.4% | 11.5% | 154.7% |

Meanwhile, structure-functional studies have reported that among the total 14 domains of ADAMTS13, the MDTCS domain remaining after removal of the CUB2 domain from TSP-2 at the C-terminus has a metalloprotease function similar to that of ADAMTS13 and is capable of cleaving VWF (Shelat et al., 2005). This suggests that only the MDTCS fragment, rather than the full-length ADAMTS13, can function as a therapeutic agent for TTP disease, and is a truncated form capable of evading neutralizing antibodies in patient plasma that bind to the C-terminus.

Accordingly, the MDTCS fragment was obtained from the selected 12 variants, and DM1 and DM2 variants having two amino acid mutations were additionally constructed by combining the selected mutant amino acid residues. According to the above-mentioned method, using the culture medium and purified solution expressed from the cells, escaping rates from single or mixed neutralizing antibodies and relative activities were measured to select final candidates.

As a result of measuring escaping rates from binding of 8 single neutralizing antibodies and relative activities using the culture medium, it was confirmed that 2B01, 3B05, 4H07, 5G08, 8D05 and DM1 evaded all neutralizing antibodies at similar levels (FIG. 28 and Table 7 below). 1C03, 2B02, 5C09, 7A02, 8D01 and DM2 showed high escaping rates from binding of 3-01 and Ab60. All of the six candidates had amino acid mutations in the domain S, and five candidates except for 8D01 had mutation at the amino acid residue at position 612. 4E11 and DM2 had high escaping rates from binding of Ab67, and both had mutation at the amino acid residue at position 314 in the domain D. DM1 had the lowest relative activity (57.9%), and 1C03 had the highest relative activity (133.1%). All candidates except for the above two candidates showed a relative activity of 78.2 to 125.1%, which is similar to that of the MDTCS-Fc control group (Table 7). In order to examine the antibody evasion ability of the 12 candidate variants except for 4C07 and 5C09 in comparison with the control MDTCS-Fc under the condition treated with 9 neutralizing antibodies (Ab3-01, Ab4-16, Ab4-20, Ab60, Ab61, Ab64, Ab65, Ab66 and Ab67) mixed together in equal proportions, 7.5 nM neutralizing antibody mixture was mixed with 4 nM culture medium in which each variant has been expressed. Then, the resulting mixture was incubated at room temperature for 1 hour, and residual activity of each variant was measured. It was confirmed that MDTCS-Fc had a residual activity of 3.5%, and 3B05, 4E11, 4H07, 5G08, and 8D05 had a residual activity of 1.24 to 2.42%, which is lower than the residual activity of MDTCS-Fc (FIG. 29). On the other hand, it was confirmed that 1C03, 2B01, 2B02, 7A02, 8D01, DM1 and DM2 had a residual activity of 7.69 to 18.81%, indicating that the ability of these candidate variants to evade the mixed neutralizing antibodies was better than MDTCS-Fc (FIG. 29).

In order to examine the neutralizing antibody evasion ability of the candidate variants using the purified solution, protein purification from each culture medium was performed using the Phytip system (protein A resin), and the single neutralizing antibody escaping rate of each variant was measured using the obtained purified solution. As a result, it was confirmed that most of the variants had a tendency similar to the results evaluated in the culture medium state (FIG. 30).

It was confirmed that the 2B01 5G08, 8D05 and DM1 variants showed a neutralizing antibody escaping rate of 24.6% or more for all of the 8 neutralizing antibodies, and 1C03, 2B02, 7A02, 8D01 and DM2 showed high escaping rates from binding of 3-01 and Ab60, similar to the results obtained using the culture medium. It was confirmed that 4E11 and DM2 showed high escaping rates from binding of Ab67 in the same manner as the results obtained using the culture medium. The relative activity of each variant is shown in FIG. 30 and Table 8 below. As a result of measuring the residual activity under the condition treated with the mixed neutralizing antibodies, it was confirmed that MDTCS-Fc had a residual activity of 3.76%, and the 3B05, 4E11, 4H07 and 8D05 variants had a residual activity of 2.05 to 3.50%, which was lower than that of MDTCS-Fc. On the other hand, it was confirmed that 1C03, 2B01, 2B02, 5G08, 7A02, 8D01, DM1 and DM2 had a residual activity of 6.34 to 12.8%, indicating that these variants had an excellent ability to evade the mixed neutralizing antibodies compared to MDTCS-Fc (FIG. 31).

Based on the above results, five candidates for additional research were selected, including 1C03, 2B02, 7A02 and DM2, selected in consideration of relative activity or escaping rate from mixed neutralizing antibodies, and 5C09 considered to have an excellent ability to evade mixed neutralizing antibodies due to the amino acid mutation at position 612.

**[Table 7]**

| Measurement of single neutralizing antibody escaping rate and relative activity of MDTCS fragments including 14 variants (culture medium condition) | | | | | | | | | | |
|---|---|---|---|---|---|---|---|---|---|---|
| **Varia nt ID** | **Mutation position** | **Neutralizing antibody escaping rate (%)** | | | | | | | | **Relative activity** |
| | | **3-01** | **4-16** | **4-20** | **Ab60** | **Ab61** | **Ab64** | **Ab65** | **Ab67** | |
| 1C03 | S612Y | 100.0% | 2.0% | -11.0% | 91.3% | 3.9% | -5.9% | -16.9% | 1.1% | 133.1% |
| 2B01 | D635V | 25.1% | 28.2% | 38.1% | 38.4% | 36.2% | 26.8% | 11.0% | 24.9% | 78.2% |
| 2B02 | R452I, S612Y | 99.0% | 1.2% | 4..2% | 93.5% | 2..9% | -2.1% | -12.9% | 3.1% | 116.5% |
| 3B05 | P618S | 35.3% | 18.9% | 25.5% | 23.6% | 19.1% | 21.3% | 17.9% | 17.8% | 92.7% |
| 4C07 | A334V | -5.9% | -11.2% | -9.6% | -11.3% | -3.3% | -12.4% | -14.6% | 14.5% | 118.2% |
| 4E11 | A314T | 15.3% | 7.3% | 11.9% | 8.1% | 9.0% | 9.7% | 8.8% | 83.5% | 89.7% |
| 4H07 | N308K | 36.1% | 20.4% | 25.8% | 19.5% | 22.5% | 25.1% | 21.0% | 53.8% | 93.6% |
| 5C09 | S612F | 100.0% | 15.7% | 18.7% | 100.0% | 12.4% | 13.0% | 0.5% | 8.5% | 108.2% |
| 5G08 | Q656H | 48.7% | 38.3% | 41.2% | 44.2% | 41.9% | 37.2% | 27.8% | 47.6% | 85.4% |
| 7A02 | S612F, G624D | 100.0% | 7.5% | 17.4% | 97.5% | 11.2% | 7.2% | -7.2% | 12.3% | 125.1% |
| 8D01 | R589Q, K608M, M609L, G624C, 1655V | 100.0% | 38.9% | 49.1% | 71.2% | 31.6% | 36.3% | 18.1% | 41.8% | 78.4% |
| 8D05 | P578L | 39.6% | 29.9% | 38.3% | 32.6% | 32.4% | 32.5% | 21.5% | 43.7% | 82.1% |
| DM1 | A314T, D635V | 52.3% | 48.2% | 56.9% | 60.6% | 54.7% | 51.6% | 38.1% | 86.8% | 57.9% |
| DM2 | A314T, S612F | 100.0% | 12.0% | 22.0% | 95.1% | 21.6% | 13.0% | 7.3% | 84.8% | 97.7% |

**[Table 8]**

| Measurement of single neutralizing antibody escaping rate and relative activity of MDTCS fragments including 14 variants (purified solution condition) | | | | | | | | | | |
|---|---|---|---|---|---|---|---|---|---|---|
| **Varian t ID** | **Mutation position** | **Neutralizing antibody escaping rate (%)** | | | | | | | | **Relative activity** |
| | | **3-01** | **4-16** | **4-20** | **Ab60** | **Ab61** | **Ab64** | **Ab65** | **Ab67** | |
| 1C03 | S612Y | 100.0% | 15.6% | 21.1% | 100.0% | 16.3% | 7.3% | -7.1% | 10.9% | 81.4%. |
| 2B01 | D635V | 46.0% | 37.8% | 49.6% | 65.5% | 43.7% | 49.7% | 28.5% | 28.2% | 56.3% |
| 2B02 | R452I, S612Y | 100.0% | 8.4% | 15.0% | 100.0% | 10.3% | 4.4% | -9.0% | 0.2% | 91.1% |
| 3B05 | P618S | 25.5% | 11.9% | 13.3% | 14.9% | 10.5% | 13.8% | 7.9% | 10.2% | 83.7% |
| 4E11 | A314T | 19.6% | 6.2% | 11.5% | 15.7% | 1.8% | 8.8% | 12.6% | 84.4% | 77.5% |
| 4H07 | N308K | 32.6% | 12.80% | 16.8% | 29.0% | 9.4% | 16.9% | 17.0% | 47.1% | 77.5% |
| 5G08 | Q656H | 89.6% | 64.5% | 70.2% | 81.9% | 57.90% | 68.9% | 59.0% | 37.7% | 18.3% |
| 7A02 | SS612F, G624D | 100.0% | 10.4% | 12.8% | 100.0% | 8.5% | 3.4% | -7.3% | 14.4% | 97.8% |
| 8D01 | RR589Q, K608M, M609L, G624C, 1655V | 100.0% | 29.5% | 48.2% | 91.7% | 20.5% | 17.1% | 14.2% | 31.5% | 65.1% |
| 8D05 | P578L | 51.0% | 27.4% | 35.1% | 49.5% | 27.2% | 33.8% | 29.9% | 46.0% | 56.3% |
| DM1 | A314T, D635V | 50.2% | 34.0% | 54.0% | 66.50% | 45.3% | 52.9% | 24.6% | 85.1% | 50.3% |
| DM2 | A314T, S612F | 100.0% | 16.2% | 24.2% | 100.0% | 24.0% | 11.5% | 11.0% | 86.5% | 73.1% |

DNA in which IgG1-YTE is conjugated to each of the finally selected 5 MDTCS variant fragments was conducted, and the escaping rate of each DNA from binding of 8 single neutralizing antibodies and the relative activity of each DNA were measured in culture medium expressed from cells. As a result, it was confirmed that 1C03, 2B02, 5C09, and 7A02 showed high escaping rates from binding of Ab3-01 and Ab60, and DM2 had high escaping rates from binding of Ab3-01, Ab60 and Ab67 (FIG. 33). The relative activity was 98.6 to 127.7%, which was similar to that of MDTCS-IgG1-YTE (FIG. 33). In order to examine the evasion ability of the 5 variants compared to the control MDTCS-IgG1-YTE under the condition treated with 9 neutralizing antibodies mixed together in equal proportions, mixed neutralizing antibodies (n=9) were mixed with the culture medium in which each variant has been expressed. Then, the resulting mixture was incubated at room temperature for 1 hour, and the residual activity of each variant was measured. It was confirmed that MDTCS-IgG1-YTE exhibited a residual activity of 5.5%, and the 5 variants exhibited a residual activity of 8.18 to 13.42%, indicating that the variants had an excellent ability to evade neutralizing antibodies compared to the control (FIG. 34).

Protein was purified from the culture medium, in which IgG1-YTE conjugated to each of the five MDTCS variant fragments has been expressed, by using the Phytip system (protein A resin). The residual activity and specific activity of neutralizing antibodies in the purified solution were measured. The concentration in the purified liquid was determined by Fc ELISA, and it was confirmed through silver staining that the target protein was eluted. As a result of measuring the residual activity under the condition treated with mixed neutralizing antibodies, it was confirmed that MDTCS-IgG1-YTE had a residual activity of 0.4%, and 1C03, 2B02, 5C09, 7A02 and DM2 exhibited residual activities of 5.7%, 1.7%, 8.8%, and 2.6%, and 7.9%, respectively, indicating that they had an excellent ability to evade the mixed neutralizing antibodies compared to MDTCS-IgG1-YTE. As a result of measuring the specific activity, it was confirmed that the four variants, except for 7A02 (10,288 IU/mg), exhibited a specific activity of 19,091 to 22,379 IU/mg, which was similar to that of the control (18,030 IU/mg) (FIG. 35). As a result, it was confirmed that the conjugate of IgG1-YTE and each of the 5 variant fragments had an excellent ability to evade the mixed neutralizing antibodies compared to the control, and that the 4 variants except 7A02 had a specific activity similar to that of the control.

### Evaluation of whether half-life of MDTCS fragment variant is increased by Fc conjugation

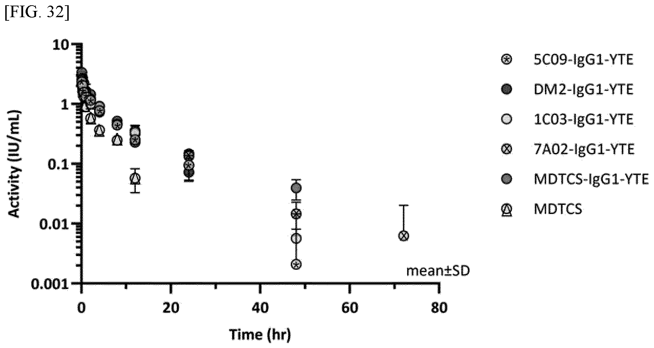

Pharmacokinetic analysis was performed in mice to confirm whether Fc (IgG1-YTE) conjugated to MDTCS actually would result in an increase in half-life. To this end, each of MDTCS, IgG1-YTE-conjugated MDTCS, and four final candidates (1C03, 5C09, 7A02, and DM2) variant fragment proteins to which IgG1-YTE has been conjugated was administered to mice through the tail vein, and plasma was collected over time. Each substance was administered to reach 160 IU/kg based on the specific activity thereof, and the activity of the substance remaining in the plasma collected at each time point was measured by activity assay. The obtained results were summarized by the naive pooled method, and pharmacokinetic analysis was performed by noncompartmental analysis using the results. It was determined that the half-life of MDTCS was 2.898 hours, the half-life of MDTCS-IgG1-YTE was 11.51 hours, and the half-life of each variant was 5.184 to 9.902 hours. The half-life was extended 1.79 to 3.97 times by IgG1-YTE conjugation compared to the control MDTCS. In addition, the mean residence rime (MRT) value representing the *in vivo* mean residence time of the candidates was 7.189 to 11.67 hours by IgG1-YTE conjugation, which increased compared to that of the control (3.743 hours) (Table 9 and FIG. 32). As a result, it was confirmed that the IgG1-YTE fusion was effective in maintaining activity by blood half-life and *in vivo* mean residence time.

**[Table 9]**

| Pharmacokinetic results | | | |
|---|---|---|---|
| Candidate | PK analysis parameters | | |
| | t_{1/2},terminal (hr) | AUC_{Inf} (IU*hr/mL) | MRT (hr) |
| MDTCS | 2.898 | 5.467 | 3.743 |
| MDTCS-IgG1-YTE | 11.51 | 16.89 | 11.67 |
| 1C03-IgG1-YTE | 6.087 | 12.33 | 8.695 |
| 5C09- IgG1- YTE | 5.184 | 12.06 | 7.982 |
| 7A02- IgG1- YTE | 9.902 | 14.49 | 10.35 |
| DM2-IgG1-YTE | 5.986 | 11.39 | 7.189 |

As described above, the present inventors have discovered 26 novel variants that evade ADAMTS13 neutralizing antibodies binding to the MDTCS portion or domain S or exhibit activity equal to or higher than that of wild-type ADAMTS13. Among these variants, 12 variants having the best ability to evade 9 neutralizing antibodies or having remarkably good relative activity were selected, and MDTCS fragments that are essential for vWF cleavage while efficiently evading neutralizing antibodies that bind to the C-terminus were constructed, thereby identifying variants having a significantly increased ability to evade autoantibodies that bind to domain D, C or S of ADAMTS13 in aTTP patients. The variant of the present invention can be advantageously used as an effective pharmacological ingredient having improved stability and long-lasting physiological activity when the blood half-life thereof is increased by IgG1-YTE conjugation.

### Evaluation of Poc for variant in aTTP mimic disease mouse model

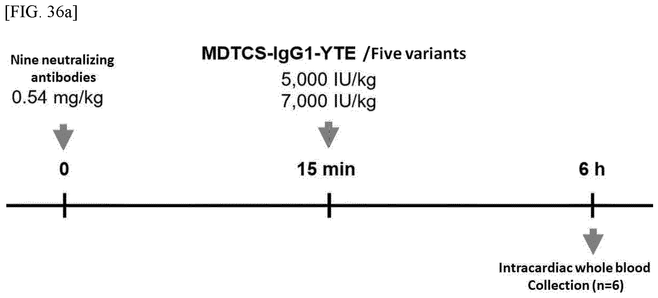

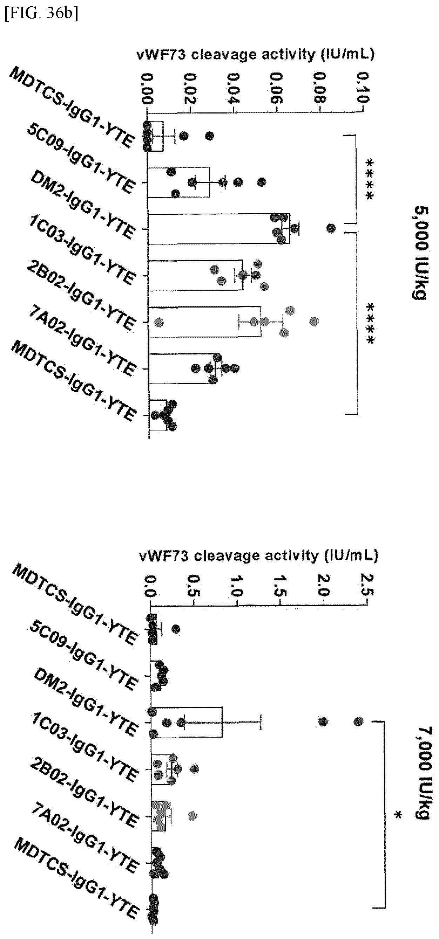

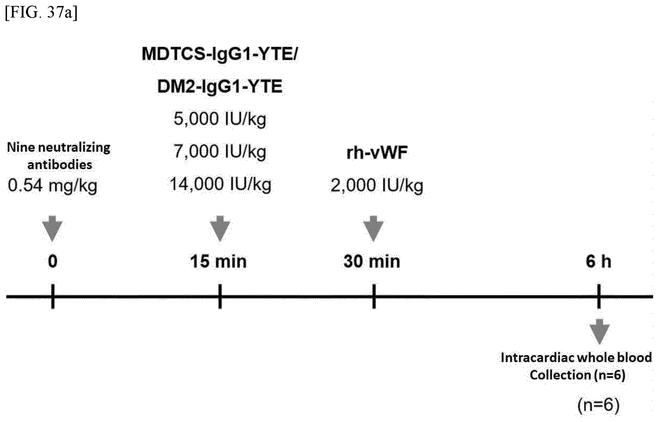

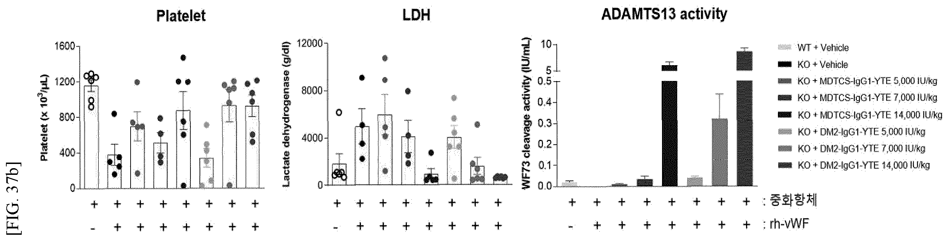

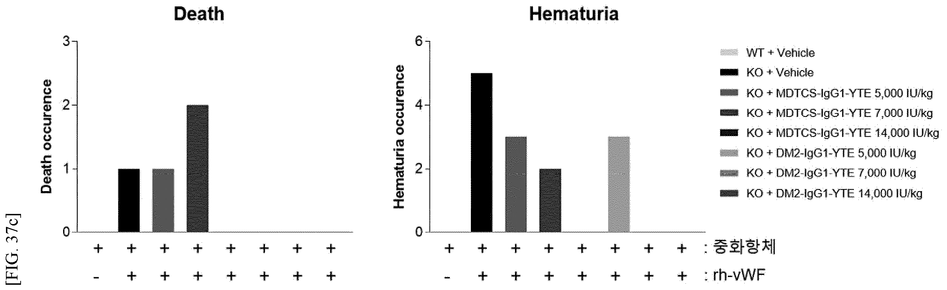

For selection of the final candidate in the established aTTP-mimic mouse model, a control substance (MDTCS-IgG1-YTE) or each of the five selected variant candidates (1C03-IgG1-YTE, 2B02-IgG1-YTE, 5C09-IgG1-YTE, 7A02-IgG1-YTE, and DM2-IgG1-YTE) was administered, and then the neutralizing antibody escaping rate thereof was evaluated (FIG. 36a). Among the five variants, DM2-IgG1-YTE exhibited the highest human ADAMTS13 activity at a dose of 5,000 or 7,000 IU/kg (FIG. 36b). The DM2-IgG1-YTE exhibiting the highest neutralizing antibody escaping rate was finally selected and administered at varying doses, and the human ADAMTS13 activity thereof and the degree of relief of clinical symptoms thereby were examined (FIG. 37a). As a result, it was confirmed that the degree of relief of clinical symptoms increased as the dose of DM2 increased, and that platelet and LDH levels were higher in the group to which DM2-IgG1-YTE was administered at a dose of 7,000 IU/kg than in the group to which MDTCS-IgG1-YTE was administered at a dose of 7,000 IU/kg (FIG. 37b). As a result of examining human ADAMTS13 activity, it could be observed that, consistent with the improvement in platelet and LDH levels, the ADAMTS13 activity increased in proportion to the dose of the control substance or the candidate substance (FIG. 37b). As a result of observing clinical symptoms, it was confirmed that death or hematuria appearing in the aTTP-mimic mouse model was alleviated by administration of the control substance or the candidate substance. In particular, in the case of the DM2-IgG1-YTE treated groups, there were no deaths at all administration doses, and no mice showing hematuria symptoms upon administration of 7,000 IU/kg or more could be observed (FIG. 37c). When 7000 IU/kg was administered, the mean residual activity of DM2-IgG1-YTE was 0.32 IU/mL, which was 9.6 times higher than that of MDTCS-IgG1-YTE 0.03 IU/mL. The difference in the above-described clinical symptom observation results was believed to be due to this difference in residual activity. As a result of general blood tests, clinical chemistry tests, and clinical symptom observation, administration of MDTCS-IgG1-YTE and DM2-IgG1-YTE trended to alleviate aTTP clinical symptoms in the aTTP-mimic mouse model, indicating *in vivo* efficacy. Particularly, it was confirmed that, when 7,000 IU/kg was administered, the alleviation effect of DM2-IgG1-YTE on aTTP clinical symptoms was better than that of MDTCS-IgG1-YTE.

### Evaluation of in vivo efficacy of variant in cTTP disease mouse model

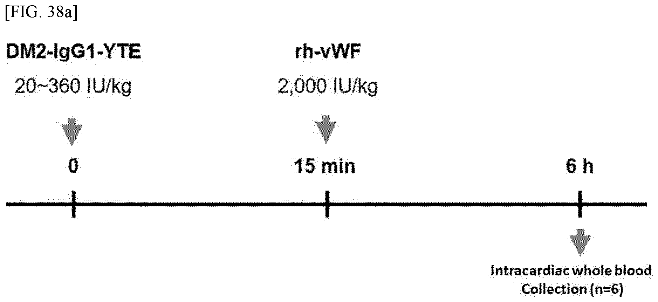

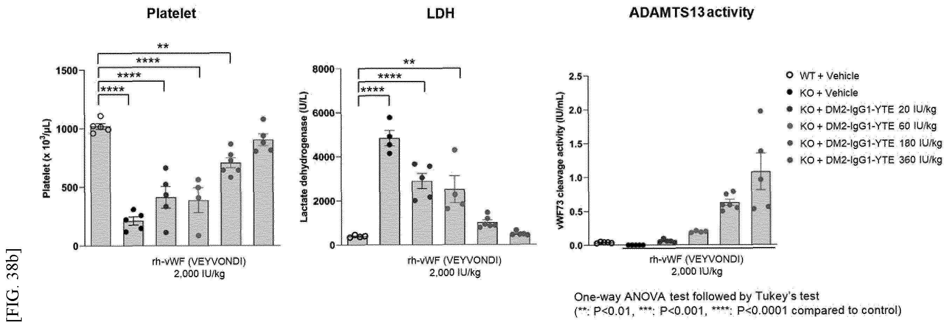

Using DM2-IgG1-YTE, which exhibited the highest efficacy among the 5 variant candidates in the aTTP mimic mouse model, the present inventors evaluated whether DM2-IgG1-YTE would alleviate hematological and clinical symptoms appearing in TTP disease in the cTTP mouse model and evaluated the recovery of human ADAMTS13 activity thereof (FIG. 38a). It could be confirmed that, as the dose of DM2-IgG1-YTE increased, platelet and LDH levels increased in a concentration-dependent manner. In the group to which DM2-IgG1-YTE 180 was administered at a dose of 360 IU/kg, there was a significant increase in platelet level compared to the control group, and in particular, it could be seen that in the group to which 360 IU/kg was administered, the platelet level was restored to the normal level (FIG. 38b). It was confirmed that the LDH level in the group to which 180 IU/kg was administered was restored to a level similar to that of the control group (FIG. 38b). In the case of ADAMTS13 activity, the groups to which DM2-IgG1-YTE was administered at a dose of 60 IU/kg or more exhibited a mean ADAMTS13 activity of 0.1 IU/mL or more, and a mean ADAMTS 13 activity of 1.08 IU/mL was measured at a dose of 360 IU/kg (FIG. 38b). Therefore, it could be seen that the DM2-IgG1-YTE variant effectively alleviates clinical symptoms and restores ADAMTS13 activity, in mice with cTTP disease. Accordingly, the present inventors performed screening and optimization of the ADAMTS13 protein formulation components as described in above Examples 1 and 2 using DM2-IgG1-YTE.

Having described a specific embodiment of the present invention, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A pharmaceutical formulation comprising 0.2 mg/ml to 1.2 mg/ml of plasma protein and 40 mM to 200 mM of amino acid stabilizer.

2. The pharmaceutical formulation of claim 1, wherein the amino acid is at least one selected from the group consisting of arginine (Arg), proline (Pro) and pharmaceutically acceptable salts thereof.

3. The pharmaceutical formulation of claim 1, further comprising a sugar stabilizer in an amount of 0 to 1.5 w/v% based on the total volume of the formulation.

4. The pharmaceutical formulation of claim 3, wherein the sugar is at least one selected from the group consisting of sucrose, trehalose, and pharmaceutically acceptable salts thereof.

5. The pharmaceutical formulation of claim 1, further comprising 100 mM to 400 mM of inorganic salt.

6. The pharmaceutical formulation of claim 5, wherein the inorganic salt is at least one selected from the group consisting of NaCl, CaCl₂, KCl and MgCl₂.

7. The pharmaceutical formulation of claim 6, wherein the inorganic salt is a mixture of NaCl and CaCl₂.

8. The pharmaceutical formulation of claim 1, further comprising a nonionic surfactant in an amount of 0.01 to 0.1 v/v% based on the total volume of the formulation.

9. The pharmaceutical formulation of claim 8, wherein the nonionic surfactant is at least one selected from the group consisting of polysorbate 80, polysorbate 60 and polysorbate 40.

10. The pharmaceutical formulation of claim 1, wherein the plasma protein is ADAMTS13 (a disintegrin and metalloproteinase with a thrombospondin type 1 motif, member 13) protein, a variant thereof, or a functional fragment thereof.

11. The pharmaceutical formulation of claim 10, wherein the variant of the ADAMTS 3 protein comprises substitution of at least one amino acid residue selected from the group consisting of residues at positions 85, 93, 126, 135, 278, 282, 308, 314, 317, 334, 364, 376, 413, 427, 452, 465, 567, 578, 585, 589, 607, 608, 609, 612, 618, 624, 630, 635, 643, 650, 651, 654, 655, 656, 658, 664 and 672 of SEQ ID NO: 1.

12. The pharmaceutical formulation of claim 11, wherein the variant of the ADAMTS13 protein is selected from the group consisting of variant proteins comprising substitution of an amino acid residue at:
positions 85 and 317; position 612; two or more of positions 282, 465 and 672; position 635; positions 452 and 612; two or more of positions 278, 334 and 427; position 618; position 135; two or more of positions 126, 567 and 651; position 413; position 334; position 314; two or more of positions 93, 364 and 376; position 308; position 656; position 607; positions 612 and 624; position 589; positions 650 and 656; position 643; positions 585 and 658; two or more of positions 630, 654 and 664; four or more of positions 589, 608, 609, 624 and 655; position 578; position 585; positions 314 and 635; and positions 314 and 612.

13. The pharmaceutical formulation of claim 11, wherein the substitution of the amino acid residue is at least one selected from the group consisting of substitution with Phe at position 85, substitution with Val at position 93, substitution with Met at position 126, substitution with Ile at position 135, substitution with Ile at position 278, substitution with Ala at position 282, substitution with Lys at position 308, substitution with Thr at position 314, substitution with His at position 317, substitution with Thr or Val at position 334, substitution with Arg at position 364, substitution with Asp at position 376, substitution with Asp at position 413, substitution with Asn at position 427, substitution with Ile at position 452, substitution with Asp at position 465, substitution with Ser at position 567, substitution with Leu at position 578, substitution with Asn or Met at position 585, substitution with Gln at position 589, substitution with Arg at position 607, substitution with Met at position 608, substitution with Leu at position 609, substitution with Phe or Tyr at position 612, substitution with Ser at position 618, substitution with Asp or Cys at position 624, substitution with Leu at position 630, substitution with Val at position 635, substitution with Phe at position 643, substitution with His at position 650, substitution with Asp at position 651, substitution with Gly at position 654, substitution with Val at position 655, substitution with Arg or His at position 656, substitution with His at position 658, substitution with Asn at position 664, and substitution with Val at position 672.

14. The pharmaceutical formulation of claim 10, wherein the plasma protein is conjugated with an Fc region derived from IgG4 immunoglobulin.

15. The pharmaceutical formulation of claim 14, wherein the Fc region comprises substitution of at least one amino acid residue selected from the group consisting of residues at positions 22, 24 and 26 of SEQ ID NO: 2.

16. The pharmaceutical formulation of claim 15, wherein the substitution of the amino acid residue is at least one selected from the group consisting of substitution with Tyr at position 22, substitution with Thr at position 24, and substitution with Glu at position 26.

17. The pharmaceutical formulation of claim 14, wherein a hinge region derived from IgG1 immunoglobulin is further comprised between the plasma protein and the Fc region derived from IgG4 immunoglobulin.

18. A composition for preventing or treating thrombotic disease comprising, as an active ingredient, the pharmaceutical formulation of any one of claims 10 to 17.

19. The composition of claim 18, wherein the thrombotic disease is thrombotic microangiopathy (TMA).

20. The composition of claim 19, wherein the thrombotic microangiopathy is selected from the group consisting of thrombocytopenic purpura (TTP), hemolytic uremic syndrome (HUS), HELLP (Hemolysis, Elevated Liver enzymes, Low Platelet count), preeclampsia, and sickle cell disease.
